(19)

(11) EP 3 170 805 B1

(12) **EUROPÄISCHE PATENTSCHRIFT**

(45) Veröffentlichungstag und Bekanntmachung des Hinweises auf die Patenterteilung:
**12.09.2018 Patentblatt 2018/37**

(21) Anmeldenummer: **15195302.3**

(22) Anmeldetag: **19.11.2015**

(51) Int Cl.:
***C07C 29/141*** *(2006.01)*
***C07C 2/24*** *(2006.01)*
***C07C 47/02*** *(2006.01)*
***C07C 69/80*** *(2006.01)*
***C07C 67/08*** *(2006.01)*
***C07C 45/50*** *(2006.01)*
***C07C 31/125*** *(2006.01)*
***C07C 11/02*** *(2006.01)*

(54) **BEEINFLUSSUNG DER VISKOSITÄT VON AUF N-BUTEN BASIERENDEN ESTERGEMISCHEN DURCH GEZIELTEN EINSATZ VON ETHEN BEI DER HERSTELLUNG DER ESTER-VORPRODUKTE**

INFLUENCING THE VISCOSITY OF N-BUTENE BASED ESTERS MIXTURES BY TARGETED USE OF ETHENE IN THE PRODUCTION OF THE ESTERS PRECURSORS

INFLUENCE DE LA VISCOSITÉ DE MÉLANGES D'ESTER À BASE DE N-BUTÈNES PAR UTILISATION CIBLÉE D'ÉTHYLÈNE LORS DE LA FABRICATION D'UN PRODUIT SEMI-FINI D'ESTER

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**24.05.2017 Patentblatt 2017/21**

(73) Patentinhaber: **Evonik Degussa GmbH**
**45128 Essen (DE)**

(72) Erfinder:
- **REEKER, Helene**
  **44227 Dortmund (DE)**
- **STOCHNIOL, Guido**
  **45721 Haltern am See (DE)**
- **MASCHMEYER, Dietrich**
  **45657 Recklinghausen (DE)**
- **ZANTHOFF, Horst-Werner**
  **45481 Mühlheim a.d. Ruhr (DE)**
- **SCHALLENBERG, Jörg**
  **46286 Dorsten (DE)**
- **GEILEN, Frank**
  **45721 Haltern am See (DE)**
- **DYBALLA, Katrin Marie**
  **45657 Recklinghausen (DE)**
- **FRIDAG, Dirk**
  **45721 Haltern am See (DE)**
- **PEITZ, Stephan**
  **45739 Oer-Erkenschwick (DE)**
- **ALTMANN, Lena**
  **45657 Recklinghausen (DE)**
- **FRANKE, Robert**
  **45772 Marl (DE)**

(56) Entgegenhaltungen:
**WO-A1-03/029180** **WO-A1-2013/168106**

EP 3 170 805 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft ein Verfahren zur Herstellung eines Estergemisches, bei welchem ein n-Buten enthaltendes Einsatzgemisch, dessen Zusammensetzung sich über die Zeit verändert, unter Erhalt eines Oligomerisats einer ersten Oligomerisierung unterworfen und zumindest ein Teil der im Oligomerisat enthaltenden Buten-Oligomere in einer Hydroformylierung in Aldehyde oxiert werden, von denen zumindest ein Teil durch anschließende Hydrierung in ein Alkoholgemisch hydriert wird, welches sodann in das Estergemisch umgesetzt wird, und bei welchem eine Näherung der Ist-Viskosität des Estergemisches bestimmt wird.

Die hier diskutierten Estergemische dienen als Weichmacher für Polyvinylchlorid (PVC). Beispiele für solche Estergemische, welche im industriell großen Maßstab produziert werden, sind Diisononyl phthalate (DINP), Benzoic acid isononyl ester (INB) und Cyclohexane-1,2-dicarboxylic acid diisononyl ester (DINCH).

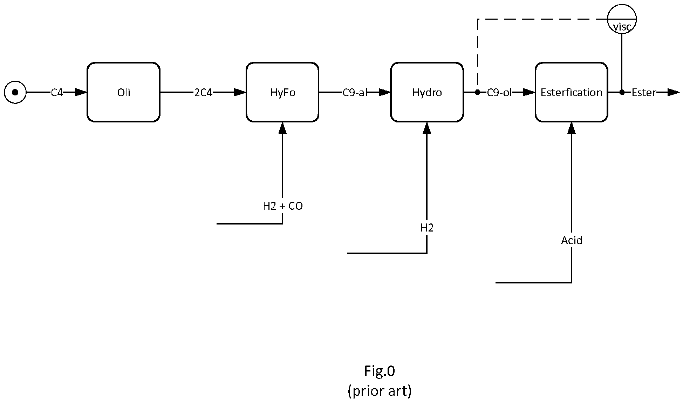

Die industrielle Produktion von DINP ist vereinfacht in Figur 0 dargestellt.

Fig. 0: Industrielle Produktion des Estergemisches DINP aus $C_4$-Rohstoffen nach dem Stand der Technik

Als Rohstoffquelle dienen Butene, genauer gesagt n-Buten. Bei den Butenen handelt es sich um Alkene (synonym: Olefine) mit vier Kohlenstoffatomen, die deswegen in der Fachsprache auch als $C_4$-Olefine bezeichnet werden. In den Figuren wird dafür die Kurzbezeichnung C4 verwendet. Es existieren die vier strukturisomeren $C_4$-Olefine Isobuten, 1-Buten, cis-2-Buten und trans-2-Buten. Isobuten ist ein verzweigtes Olefin, die drei linearen $C_4$-Oelfine 1-Buten, cis-2-Buten und trans-2-Buten werden oft als n-Buten zusammengefasst. n-Buten ist also als ein Gemisch aus linearen Butenen zu verstehen und enthält mindestens eine der drei Substanzen 1-Buten, cis-2-Buten und trans-2-Buten, aber nicht zwangsläufig alle diese drei Substanzen. So kann beispielsweise auch ein Gemisch aus den beiden 2-Butenen, welches frei ist von 1-Buten, als n-Buten verstanden werden.

$C_4$-Olefine fallen u.a. beim Steamcracken von Naphtha an (so genanntes Crack-C4). Aufgrund von Veränderungen in der Rohstoffbasis ist die Verfügbarkeit von Crack-C4 in den vergangenen Jahren zurückgegangen, sodass zunehmend andere $C_4$-Quellen erschlossen werden, etwa aus Fluid-katalytischen Naphtha-Cracken (FCC-C4) oder aus der Dehydrierung von Butan.

Die genaue Zusammensetzung des n-Butens, welches als Ausgangsstoff für die Herstellung des Estergemisches eingesetzt wird, hängt stark von der verfügbaren Rohstoffquelle ab. Zudem hat die vorgelagerte Aufarbeitung des $C_4$-Rohstoffes, in der noch andere Wertstoffe wie 1,3-Butadien, Isobuten und 1-Buten abgeschöpft und Störstoffe entfernt werden, Einfluss auf die Zusammensetzung des n-Butens.

Eine Einführung in die Chemie der $C_4$-Oelfine und deren industrielle Nutzung findet sich in:
Geilen, F. M., Stochniol, G., Peitz, S. and Schulte-Koerne, E.: Butenes. Ullmann's Encyclopedia of Industrial Chemistry. Published Online: 31 JAN 2014 DOI: 10.1002/14356007.a04_483.pub3

Bei der Herstellung von DINP werden in einem ersten Reaktionsschritt die linearen Butene einer Oligomerisierung (Oli) unterworfen.

Unter der Oligomerisierung versteht man die Reaktion von Kohlenwasserstoffen mit sich selbst, wobei entsprechend längerkettige Kohlenwasserstoffe entstehen. So lässt sich beispielsweise durch die Oligomerisierung von zwei Olefinen mit drei Kohlenstoffatomen (Dimerisierung) ein Olefin mit sechs Kohlenstoffatomen aufbauen. Verbinden sich hingegen drei Olefine mit drei Kohlenstoffatomen miteinander (Trimerisierung), entsteht ein Olefin mit neun Kohlenstoffatomen.

Werden Butene einer Oligomerisierung unterworfen, entstehen dabei im Wesentlichen Olefine mit acht Kohlenstoffatomen ($C_8$-Olefine, oft "Dibutene" genannt), Olefine mit zwölf Kohlenstoffatomen ($C_{12}$-Olefine, "Tributene") sowie in einem geringeren Umfang Olefine mit mehr als zwölf Kohlenstoffatomen ($C_{12+}$-Olefine).

Das Produkt der Oligomerisierung, das so genannte Oligomerisat, enthält also unterschiedliche Buten-Oligomere, nämlich Dibutene, Tributene, höhere Oligomere sowie auch nicht umgesetzte Butene. Für den hier geschilderten Produktionsprozess sind nur die Dibutene (2C4) von Interesse. Sie werden aus dem Oligomerisat destillativ abgetrennt. Der Einfachheit halber ist dies aber nicht in den Figuren dargestellt.

Einzelheiten der industriell praktizierten Oligomerisierung von $C_4$-Olefinen finden sich in der Nicht-Patentliteratur, beispielsweise unter:
R. H. Friedlander, D. J. Ward, F. Obenaus, F. Nierlich, J. Neumeister: Make plasticizer olefins via n-butene dimerization. Hydrocarbon Processing, February 1986, Seiten 31 bis 33.

Innerhalb der Patentliteratur beschreibt beispielsweise DE102008007081A1 eine Oligomerisierung von Butenen. Die EP1029839A1 befasst sich mit der Fraktionierung des Oligomerisats in die einzelnen Buten-Oligomere.

Auf die Oligomerisierung folgt als zweiter Reaktionsschritt eine Hydroformylierung (HyFo). Dabei werden die Dibutene (2C4) mit Synthesegas (H2+CO) - das ist eine Mischung aus Wasserstoff ($H_2$) und Kohlenmonoxid (CO) - in ein Gemisch aus Aldehyden mit neun Kohlenstoffatomen (C9-al) umgesetzt.

Eine gute Übersicht über den allgemeinen Stand der Hydroformylierung von Olefinen findet sich in

R. Franke, D. Selent, A. Börner, "Applied Hydroformylation", Chem. Rev., 2012 (112), S. 5675-5732, DOI:10.1021/cr3001803.

Bei der Hydroformylierung entstehen neben den gewünschten Aldehyden (C9-al) auch unerwünschte Nebenprodukte, die aus dem Reaktionsgemisch der Hydroformylierung abgetrennt werden müssen. Die Aufarbeitung von Hydroformylierungsgemischen ist nicht trivial; vgl. WO2014131623. Da es hier nicht darauf ankommt, wie die Aldehyde aus dem Reaktionsgemisch der Hydroformylierung gewonnen werden, ist dies in den Figuren nicht dargestellt.

Die $C_9$-Aldehyde werden sodann in einem dritten Reaktionsschritt zu Alkoholen mit neun Kohlenstoffatomen (C9-ol) hydriert. Bei der Hydrierung (Hydro) wird Wasserstoff ($H_2$) an den Aldehyd angelagert, sodass ein Alkohol entsteht. Da hier ein Gemisch unterschiedlicher Aldehyde mit neun Kohlenstoffatomen (C9-al) hydriert wird, liegt nach der Hydrierung ein Gemisch aus verschiedenen Alkoholen mit neun Kohlenstoffatomen (C9-ol) vor, welches Isononanol, kurz INA, genannt wird. Isononanol ist ein Gemisch aus isomeren Nonylalkoholen wie zum Beispiel n-Nonanol und einfach und/oder mehrfach verzweigten Nonanolen wie insbesondere Methyloctanol. Eine näher gehende Beschreibung der Hydrierung von durch Hydroformylierung hergestellten Aldehyden findet sich in EP1219584B1.

In einem letzten Produktionsschritt, der Veresterung (Esterfication) wird das Alkoholgemisch (C9-ol) zu einem Estergemisch (Ester) umgesetzt. Ester werden allgemein durch Zugabe von Säure zu Alkoholen oder durch Umesterung eines bestehenden Esters in Gegenwart eines Alkohols gebildet.

Im industriellen Format wird die Nonanolmischung mit Phthalsäure oder Phthalsäureanhydrid (Acid) verestert. Das als Weichmacher eingesetzte Estergemisch ist dann das Diisononylphthalat (DINP). Dieses kann prinzipiell auch durch Umesterung eines Dialkylphthalats mit Isononylalkohol hergestellt werden, Verwendung findet in dem Zusammenhang vor allem Dimethylphthalat.

Einzelheiten zu solchen Veresterungsverfahren können EP1186593B1 und EP1300388B1 entnommen werden.

Für die Produktqualität des hergestellten Weichmachers ist die Viskosität des Estergemisches maßgeblich. Abhängig von dem Einsatzzweck des weichgemachten PVC ist entweder eine hohe Viskosität oder eine niedrige Viskosität erforderlich. In beiden Fällen wünschen sich die Verarbeiter von Weichmachern eine möglichst konstante Viskosität der eingesetzten Estermischungen über lange Produktionszeiträume hinweg, da einmal festgelegte Rezepturen ungern an schwankende Produktqualitäten angepasst werden wollen. Die Weichmacherhersteller sind deswegen dazu aufgerufen, ihre Estergemische über lange Zeiträume hinweg mit konstanter Viskosität zu liefern.

Zum Zwecke der Qualitätssicherung wird die Viskosität des produzierten Estergemisches (Ester) mit einem Instrument (visc) gemessen, sodass unter Berücksichtigung der Messfehler eine Näherung der Ist-Viskosität des Estergemisches erhalten wird.

Die Viskosität der Estergemische wird maßgeblich durch die Isomerenverteilung des zu veresternden Alkoholgemisches bestimmt. Dieser Zusammenhang wird in EP1430014B1 aufgezeigt und sogar eine Formel angegeben, nach der sich die Viskosität des späteren Estergemisches aus der Analytik des veresterten Alkoholgemisches berechnen lässt. In Figur 0 deutet die gestrichelte Linie zwischen dem Austrag der Hydrierung (C9-ol) und dem Instrument (visc) eine solche rechnerische Bestimmung der Näherung der Ist-Viskosität des Estergemisches auf Grundlage der Zusammensetzung der Alkohole an.

Die Zusammensetzung des Alkoholgemisches hängt wiederum von der Isomerenverteilung der hydroformylierten Buten-Oligomere ab. Ein Maß für die Isomerenverteilung von Buten-Oligomeren ist der Verzweigungsgrad, gemessen als Isoindex; vgl. US6433242B1.

Der Verzweigungsgrad der Buten-Oligomere ergibt sich wiederum aus der Zusammensetzung der Einsatzstoffe der Oligomerisierung: So führt ein $C_4$-Olefingemisch, welches besonders reich an 1-Buten ist, zu wenig verzweigten Buten-Oligomeren, während solche $C_4$-Einsatzstoffe, die überwiegend aus cis-2-Buten und trans-2-Buten bestehen, ohne weitere Maßnahmen zu hohen Isoindizies führen; vgl. WO2014/207034A1.

Letztendlich ist also bei dem in Figur 0 dargestellten Produktionsablauf die Zusammensetzung der eingesetzten $C_4$-Olefine für die Viskosität des hergestellten Estergemisches verantwortlich.

Solange die Quelle der $C_4$-Olefine eine verlässliche Qualität liefert - also ein $C_4$-Gemisch mit einer zeitlich konstanten Zusammensetzung - und die Verarbeitungsprozesse beherrscht werden, gelingt es Estergemische mit konstanter Viskosität herzustellen.

Wenn nun aber die unstetige Quelle genutzt werden soll, die $C_4$-Gemische liefert, deren Zusammensetzung sich laufend ändert, gelingt es mit herkömmlicher Prozessführung kaum noch, eine konstante Weichmacherqualität zu erzielen.

Da verlässliche und hochwertige $C_4$-Quellen wie Naphtha-Steamcracker immer seltener werden, weichen die Hersteller $C_4$-basierter Produkte zunehmend auf alternative Rohstoffquellen aus, die verglichen mit Crack-C4 eine minderwertige Zusammensetzung aufweisen.

So beschreibt etwa die WO2014/207034A1 wie sich aus einem minderwertigen $C_4$-Gemisch, welches kaum 1-Buten enthält, noch für die Weichmacherherstellung geeignete Oligomerisate machen lassen.

Ein weiterer Nachteil alternativer $C_4$-Quellen ist, dass die Zusammensetzung der gelieferten Rohstoffströme starken zeitlichen Änderungen unterworfen ist. Insbesondere dann, wenn sich der die Viskosität stark beeinflussende Gehalt an 1-Buten ständig ändert, ist es schwierig, eine gleichbleibende Weichmacherqualität zu gewährleisten.

In der zum Anmeldezeitpunkt noch unveröffentlichten Anmeldung EP 15168100.4 bzw. US 14/717,183 sind technische Maßnahmen beschrieben, die eine zeitlich veränderliche $C_4$-Quelle für die Herstellung von Weichmacherestern nutzbar machen. Diese Maßnahmen zielen allesamt auf die Verfahrensführung ab und sind technisch sehr aufwändig.

Nach alledem liegt der Erfindung die Aufgabe zu Grunde, ein vergleichsweise einfaches Verfahren anzugeben, welches es ermöglicht, ein n-Buten mit zeitlich veränderlicher Zusammensetzung in ein Estergemisch umzusetzen, dessen Viskosität selbst dann über einen langen Zeitraum weitestgehend konstant gehalten werden kann, wenn eine unstetige $C_4$-Quelle genutzt wird, die über diesen Zeitraum schwankende Qualitäten liefert.

Überraschenderweise gelingt dies durch gezielten Einsatz eines zweiten Rohstoffes, nämlich Ethen. Es wurde gefunden, dass die Viskosität von auf n-Buten basierenden Estergemischen durch gezielten Einsatz von Ethen bei der Herstellung der Ester-Vorprodukte beeinflusst werden kann.

Gegenstand der Erfindung ist mithin die Beeinflussung der Viskosität von auf n-Buten basierenden Estergemischen durch gezielten Einsatz von Ethen bei der Herstellung der Ester-Vorprodukte.

Konkret schlägt die Erfindung zwei Maßnahmen vor, wie das Ethen eingesetzt wird, nämlich einmal als $C_2$-Olefin direkt und einmal als $C_8$-Olefin nach vorhergehender separater Oligomerisierung.

In einer ersten Ausführungsform der Erfindung werden in Abhängigkeit von der bestimmten Näherung der Ist-Viskosität des Estergemisches den zu oxierenden Buten-Oligomeren gezielt Ethen-Oligomere zugegeben, welche dadurch erhalten sind, dass ein von n-Buten freies, Ethen enthaltendes Einsatzgemisch einer von der ersten Oligomerisierung separat durchgeführten zweiten Oligomerisierung unterworfen wird. In der zweiten Oligomerisierung wird Ethen unter anderem tetramerisiert, sodass $C_8$-Olefine anfallen, die durch Verbindung von vier $C_2$-Olefinen entstehen. Bei der Oligomerisierung von Ethen fallen neben den $C_8$-Olefinen auch $C_4$-, $C_6$-, und höhere-Olefine an, die aber nicht alle mit dem Oligomerisat der Buten-Oligomerisierung vermischt werden müssen. Wenn die Hydroformylierung auf die Herstellung von $C_m$-Aldehyden abzielt, werden die $C_{m-1}$ Olefine aus dem Oligomerisat der Ethen Oligomerisierung abgetrennt und mit dem Oligomerisat der Buten Oligomerisierung vermischt. Gilt es also $C_9$-Aldehyde in der Hydroformylierung herzustellen, werden die Tetramere aus der $C_2$-Oligomerisierung mit den $C_8$-Olefinen aus der $C_4$-Oligomerisierung (Dibuten) vermischt. Das so erhaltene Gemisch beinhaltet somit zwei Arten von $C_8$-Olefinen, nämlich einerseits Buten-Dimere und andererseits Ethen-Tetramere. Dieses Gemisch von $C_8$-Olefinen unterschiedlicher Genese wird sodann der Hydroformylierung unterzogen.

Der Effekt der Zumischung des $C_2$-basierten Oligomerisats besteht darin, dass die Tetramere des Ethens eine geringere Verzweigung aufweisen als die Dimere des Butens, und dass somit durch die Vermischung der minimal verzweigten $C_2$-Oligomere und der höher verzweigten $C_4$-Oligomere insgesamt ein $C_8$-Olefingemisch mit einer geringeren Verzweigung entsteht. Dieses führt wiederum zu einer geringeren Viskosität des daraus hergestellten Estergemisches.

Um die Viskosität des späteren Estergemisches zu senken, ist demgemäß der Anteil an Ethen-Oligomeren in dem Cs-Gemisch relativ zu den Buten-Oligomeren zu erhöhen. Ist umgekehrt die Viskosität zu erhöhen, wird die Zugabe von Ethen-Tetrameren zu dem zu oxierenden Substrat verringert.

In einer zweiten Ausführungsform der Erfindung wird in Abhängigkeit von der bestimmten Näherung der Ist-Viskosität des Estergemisches dem n-Buten enthaltenden Einsatzgemisch gezielt Ethen zugegeben, sodass es sich bei der ersten Oligomerisierung um eine Co-Oligomerisierung von Ethen und n-Buten handelt. Co-Oligomerisierung bedeutet in diesem Zusammenhang, dass das Ethen und das Buten gleichzeitig in demselben Reaktionsgefäß an demselben Katalysator oligomerisiert werden. Die Vermischung der Ethen-Oligomere und der Buten-Oligomere erfolgt dann in situ im Reaktor und nicht erst durch Abmischen der separat erhaltenen Oligomerisate. Gleichzeitig bilden sich im Reaktor auch Oligomerisate, die sowohl Buten als auch Ethen enthalten. So können C8-Olefine gebildet werden sowohl aus zwei Butenmolekülen, oder aus vier Ethenmolekülen, aber auch aus zwei Ethenmolekülen und einem Butenmolekül.

Der Effekt auf die Viskosität ist derselbe: Je hoher der Anteil an Ethen in der Co-Oligomerisierung, desto niedriger die Viskosität der späteren Ester. Die Steuerung erfolgt dann entsprechend über den Grad der Zumischung des Ethens zu dem Buten enthaltenden Einsatzstrom.

Die beide Ausführungsformen verbindende Grundidee der Erfindung besteht somit darin, das schwankende $C_4$-Einsatzgemisch bzw. die daraus abgeleiteten Buten-Oligomere gezielt aufzuwerten durch Zugabe von Ethen bzw. Ethen-Oligomeren, da sich auf diese Weise die resultierende Viskosität des Estergemisches beeinflussen lässt.

Der besondere Vorteil dieses Verfahrens ist darin zu sehen, dass die eigentlichen Produktionsprozesse kaum verändert werden, sondern stattdessen Einfluss auf die verarbeiteten Stoffe genommen wird. Insoweit unterscheidet sich die Erfindung von den in EP 15168100.4 bzw. US 14/717,183 beschriebenen Maßnahmen, welche keinen Einfluss auf die Stoffströme nehmen sondern aufwändig Anlagenparameter ändern.

Ein Prinzip bedingter Nachteil dieses Verfahrens ist freilich darin zu sehen, dass es auf eine zweite Rohstoffquelle angewiesen ist, welche das $C_2$-Olefin Ethen liefert, und dies möglichst in gleichbleibender Qualität.

Allerdings ist Ethen eine gut verfügbare Petro-Chemikalie, da sie in großen Mengen für die Herstellung von Polyethylen benötigt wird. Außerdem lässt sich Ethen aus unkonventionellen Erdgasvorkommen gewinnen. Mithin gibt es vielfältige Möglichkeiten an das für die Durchführung des Verfahrens benötigte Ethen zu gelangen. Die mit der $C_2$-Quelle verbundenen Mehrkosten werden dadurch kompensiert, dass das Verfahren eine minderwertige $C_4$-Quelle nutzen kann, die deutlich preiswerter ist als die bisher genutzten hochwertigen und damit teuren $C_4$-Quellen.

In einer ersten Weiterbildung der Variante der Erfindung, in der Ethen-Oligomere dem Oligomerisat des n-Buten enthaltenden Einsatzgemisches zugegeben werden, werden die Ethen-Oligomere dadurch erhalten, dass ein von n-Buten freies, Ethen und Hexen enthaltendes Einsatzgemisch der von der ersten Oligomerisierung separat durchgeführten zweiten Oligomerisierung unterworfen wird, sodass es sich bei der zweiten Oligomerisierung um eine Co-Oligomerisierung von Ethen und Hexen handelt. Hexen versteht sich in diesem Zusammenhang als Sammelbegriff für eines oder mehrere Olefine mit sechs Kohlenstoffatomen wie 1-Hexen oder n-Hexen. Die Co-Oligomerisierung von Ethen und dem $C_6$-Olefin hat den Effekt, dass das Hexen als Lösungsmittel für das Ethen fungiert, wodurch die Reaktionsbedingungen besser kontrolliert werden können. Dieser Effekt ist in der zum Zeitpunkt der Anmeldung noch unveröffentlichten europäischen Patentanmeldung 15151621.8 beschrieben.

Es empfiehlt sich, das Lösungsmittel Hexen von den gewünschten Ethen-basierten $C_8$-Oligomeren abzutrennen, bevor diese mit dem Dibuten vermischt werden. Andernfalls würde man Ballast mitschleppen, der in den späteren Stufen zu Nebenreaktionen führt und die Qualität des Esters mindern kann.

Ein wichtiger technologischer Vorteil der Co-Oligomerisierung von Ethen mit Hexen ist, dass diese in der flüssigen Phase erfolgen kann. Die Oligomerisierung ist nämlich stark exotherm und in dem Hexen als flüssigem Lösungsmittel lässt sich die Reaktionswärme leicht abführen. Die Buten-Oligomerisierung findet auch in der flüssigen Phase statt. Ein weiterer Vorteil der flüssigen Ethen-Oligomerisierung ist, dass für diese die gleichen Reaktoren und Anlagenteilen verwendet werden können wie bei der Buten-Oligomerisierung, so dass die Komplexität der Anlage klein gehalten wird.

Hexen ist als isolierter Rohstoff kaum erhältlich. Es kann aber aus den üblichen $C_2$- und $C_3$-Quellen durch Oligomerisierung gewonnen werden. Mithin wird das Hexen aus einer von der ersten und zweiten Oligomerisierung separat durchgeführten dritten Oligomerisierung bezogen, in welcher Ethen trimerisiert wird oder in welcher Propen dimerisiert wird.

Anstatt das Hexen separat herzustellen, kann es auch aus der ohnehin vorhandenen Ethen-Oligomerisierung bezogen werden: In der zweiten Oligomerisierung, die immer Ethen enthält, entsteht auch immer Hexen, unabhängig davon, ob noch ein anderes Olefin oder ein Lösungsmittel vorhanden ist. Mithin fällt Hexen in der ersten Ausführungsform als Nebenprodukt in der Ethen-Tetramerisierung, und in der zweiten Ausführungsform als Nebenprodukt der Co-Oligomerisierung von n-Buten mit Ethen an. Eine Variante des Verfahrens besteht mithin darin, dass das Hexen aus der zweiten Oligomerisierung bezogen wird.

Der Vorteil der Co-Oligomerisierung von Ethen und Hexen besteht darin, dass die ohnehin als Nebenprodukt anfallenden Trimere des Ethens sinnvoll als Lösungsmittel verwendet werden können. Zudem ermöglicht das Hexen als Lösungsmittel im Gegensatz zu anderen denkbaren Lösungsmitteln wie z. B. Isobutan günstigere Bedingungen bezüglich der destillativen Abtrennung, da nicht so große Mengen an Lösungsmittel unter erhöhten Drücken über Kopf abgenommen werden müssen. Damit sinken die Kosten für Installation und Betrieb der Lösungsmittel-Abtrennung.

Eine bevorzugte Weiterbildung der Ausführungsform des Verfahrens, in der Ethen und Buten separat oligomerisiert werden, besteht darin, die Ethen-Oligomerisierung in Gegenwart eines inerten, flüssigen Lösungsmittels durchzuführen. Das Ethen wird dann zumindest teilweise in dem flüssigen Lösungsmittel gelöst. Die Ethen-Oligomere liegen dann auch in dem Lösungsmittel vor und sollten vor Vermischen mit den Buten-Oligomeren abgetrennt werden. Das Lösungsmittel kann dann rezykliert und mit frischem Ethen vermischt werden. Der Vorteil der Oligomerisierung in Gegenwart des inerten Lösungsmittels besteht darin, dass sich die Reaktionsbedingungen besser kontrollieren lassen. Außerdem ist es so einfacher, das leicht flüchtige Ethen in der flüssigen Phase an einem festen heterogenen Katalysator zu oligomerisieren und damit eine hohe Prozessintensität zu erzielen. Als inerte Lösungsmittel kommen solche Stoffe in Betracht, die so reaktionsträge sind, dass sie an der Oligomerisierung nicht teilnehmen. Konkret können das Propan, Isobutan, Pentan, Hexan oder Heptan oder Mischungen dieser Alkane sein. Allerdings sollten nur solche Alkane vermischt werden, die entweder einen höheren oder niedrigeren Siedepunkt als die Ethen-Oligomere haben, sodass die Abtrennung der Ethen-Oligomere von dem Lösungsmittel nicht zu kompliziert wird.

Im Übrigen kann es auch vorteilhaft sein, ein inertes Lösungsmittel bei der $C_2$-Oligomerisierung einzusetzen, wenn diese in Gegenwart von reaktivem Hexen erfolgt.

In einer bevorzugten Weiterbildung der Ausführungsform, in der Ethen und Buten separat oligomerisiert werden, erfolgt die gezielte Zugabe der Ethen-Oligomere zu den Buten-Oligomeren dergestalt, dass zunächst eine Soll-Viskosität des Estergemisches festgelegt wird, dass die bestimmte Näherung der Ist-Viskosität des Estergemisches mit der Soll-Viskosität des Estergemisches verglichen wird, und dass die Menge der den zu oxierenden Buten-Oligomeren zugegebenen Ethen-Oligomere erhöht wird, wenn die Näherung der Ist-Viskosität größer ist als die Soll-Viskosität bzw. dass die Menge der den zu oxierenden Buten-Oligomeren zugegebenen Ethen-Oligomere verringert wird, wenn die Näherung der Ist-Viskosität kleiner ist als die Soll-Viskosität.

Dementsprechend erfolgt bei der Ausführungsform, in der Ethen und Buten co-oligomerisiert werden, die gezielte Zugabe des Ethens zum Einsatzgemisch dergestalt, dass zunächst eine Soll-Viskosität des Estergemisches festgelegt wird, dass die bestimmte Näherung der Ist-Viskosität des Estergemisches mit der Soll-Viskosität des Estergemisches verglichen wird, und dass die Menge des dem Einsatzgemisch zugegebenen Ethens erhöht wird, wenn die Näherung der Ist-Viskosität größer ist als die Soll-Viskosität bzw. dass die Menge des dem Einsatzgemisch zugegebenen Ethens verringert wird, wenn die Näherung der Ist-Viskosität kleiner ist als die Soll-Viskosität. Dieser Steuerungsansatz beruht auf der Erkenntnis, dass mit steigendem Ethen-Anteil die Viskosität sinkt und umgekehrt.

Die Näherung der Ist-Viskosität des Estergemisches wird benötigt, um abschätzen zu können, ob der Ethen-bzw. der Ethen-Oligomeranteil gesenkt oder gesteigert werden muss, um letztendlich die Viskosität des Estergemisches zu verstetigen. Im einfachsten Fall erfolgt die Bestimmung der Näherung der Ist-Viskosität des Estergemisches durch Messung.

Für welche Ist-Viskosität nach welcher Messmethode unter welchen Bedingungen die Näherung bestimmt wird, ist für die Verwirklichung der Erfindung unerheblich. Die Soll-Viskosität wird letztendlich sowohl hinsichtlich ihrer Größe als auch hinsichtlich ihrer Messmethode von den Kunden bestimmt, die das Estergemisch einsetzen. Die Näherung der Ist-Viskosität wird mithin nach der Methode bestimmt, die für die Soll-Viskosität vorgeben ist.

So ist es beispielsweise möglich, die Näherung der Ist-Viskosität nach ASTM D7042 zu bestimmen. Im Rahmen der Messgenauigkeit entspricht dann die Näherung der Ist-Viskosität der tatsächlichen Ist-Viskosität.

Die Messung der Ist-Viskosität des Estergemisches setzt voraus, dass der letzte Verfahrensschritt (die Veresterung) schon abgeschlossen ist. In der industriellen Praxis erfolgt die Veresterung aber in der Regel batch-weise, während die übrigen, vorgelagerten Reaktionsschritte kontinuierlich durchgeführt werden. Dies bedeutet, dass einige Zeit vergeht, bis die Veresterung abgeschlossen ist und die erzielte Viskosität gemessen werden kann. In der Zwischenzeit laufen aber Oligomerisierung, Hydroformylierung und Hydrierung kontinuierlich weiter und auf die Isomerenverteilung des zwischenzeitlich produzierten Alkoholgemisches kann kein Einfluss mehr genommen werden, wenn die Messung eine unakzeptable Abweichung von der Soll-Viskosität ergeben sollte: Durch die batch-weise Veresterung wird eine Totzeit hervorgerufen, welche die Regelungsgüte deutlich schmälert.

Um dieses Problem vermeiden, schlägt die Erfindung stattdessen vor, die Näherung der Ist-Viskosität durch Berechnung zu bestimmen, wobei die Berechnung auf Grundlage einer Analytik der Alkohole erfolgt. Ein geeignetes Berechnungsverfahren, nach dem sich aus der Zusammensetzung der Alkohole die Viskosität des späteren Estergemisches bestimmen lässt, ist in EP1430014B1 offenbart.

Ein besonderer Vorteil der rechnerischen Bestimmung der Näherung der Ist-Viskosität über obige Formel besteht darin, dass die Analytik der Alkohole automatisiert und kontinuierlich erfolgen kann; nämlich durch Gaschromatographie. Mithin kann die Viskosität ebenfalls kontinuierlich und praktisch ohne Totzeit bestimmt werden. Auf diese Weise ist es möglich, auf Schwankungen in der Zusammensetzung der Butene frühzeitig durch Ethen- bzw. Ethen-Oligomer-Zugabe zu reagieren. Die Regelungsgüte wird erheblich verbessert.

Alternativ kann die Näherung der Ist-Viskosität des Estergemisches durch Berechnung bestimmt werden, wobei die Berechnung auf Grundlage einer Analytik des Aldehydgemisches erfolgt. Die Berechnung erfolgt analog zu dem in EP1430014B1 beschrieben Verfahren.

Ebenso alternativ kann die Näherung der Ist-Viskosität des Estergemisches durch Berechnung auf Grundlage einer Analytik der zu oxierenden Oligomere erfolgen. Bei der Bestimmung der Näherung der Ist-Viskosität müssen alle $C_8$-Isomeren berücksichtigt werden; auch solche, die aus Ethen stammen bzw. durch Verbindung von $C_2$ mit $C_2$ mit $C_4$ oder aus Verbindung von $C_2$ mit $C_6$ entstanden sind. Die Berechnung erfolgt analog zu dem in EP1430014B1 beschrieben Verfahren.

Das vorliegende Verfahren ist bestimmt zur Verwertung von $C_4$-Quellen, welche kontinuierlich einen Rohstoffstrom liefern, der zeitlichen Schwankungen hinsichtlich seiner Zusammensetzung unterliegt. Eine solche unstetige $C_4$-Quelle liefert typischerweise ein n-Buten-haltiges Einsatzgemisch, welches sich zusammensetzt aus einer Kombination der folgenden Substanzmassenströme, die sich innerhalb der angegebenen Substanzmassenstrombereiche mit einer jeweiligen, im angegebenen Veränderungsgeschwindigkeitsbereich liegenden Veränderungsgeschwindigkeit verändern:

| Substanz | Substanzmassenstrom | Veränderungsgeschwindigkeit |
|---|---|---|
| Isobuten: | 0 kg/s bis 1 kg/s | - 0.05 $g/s^2$ bis 0.05 $g/s^2$ |
| 1-Buten: | 0 kg/s bis 6 kg/s | - 0.30 $g/s^2$ bis 0.30 $g/s^2$ |
| 2-Buten (cis + trans): | 1 kg/s bis 13 kg/s | - 0.30 $g/s^2$ bis 0.30 $g/s^2$ |
| Isobutan: | 0 kg/s bis 3 kg/s | - 0.15 $g/s^2$ bis 0.15 $g/s^2$ |
| n-Butan: | 1 kg/s bis 7 kg/s | - 0.30 $g/s^2$ bis 0.30 $g/s^2$ |

(fortgesetzt)

| Substanz | Substanzmassenstrom | Veränderungsgeschwindigkeit |
|---|---|---|
| Sonstige Stoffe: | 0 kg/s bis 1 kg/s | - 0.05 $g/s^2$ bis 0.05 $g/s^2$ |

Die Veränderungsgeschwindigkeit versteht sich mathematisch als das zeitliche Differenzial des Substanzmassenstroms.

So könnte ein spezifikationsgemäßer Strom des Einsatzgemisches pro Sekunde 1 kg 1-Buten liefern, wobei sich dieser Wert mit einer Geschwindigkeit von 0.25 $g/s^2$ verändern dürfte. Dies bedeutet, dass innerhalb von 100 000 Sekunden (= 28 Stunden) der Teilmassenstrom des 1-Buten auf bis zu 3.5 kg/s anschwillt. Umgekehrt ist auch eine Verringerung des 1-Buten-Gehaltes mit einer Veränderungsgeschwindigkeit von -0.1 $g/s^2$ spezifikationsgerecht, sodass ein 1 kg/s 1-Buten-Strom innerhalb von weniger als drei Stunden (nämlich in 10 000 Sekunden) vollständig versiegt, sodass der $C_4$-Strom plötzlich frei von 1-Buten ist. Da sämtliche Komponenten zeitlichen Schwankungen unterliegen, kann es auch zu Schwankungen des Gesamtmassenstromes kommen. Diese Schwankungen werden limitiert durch die Grenzen des Betriebsbereichs der jeweiligen Anlage.

Um diese Schwankungen auszugleichen, wird gemäß der Erfindung bei der Herstellung der Ester-Vorprodukte Ethen eingesetzt. Dieses Ethen muss dann aber kontinuierlich und mit geringen Schwankungen bereitgestellt werden, sodass sich die Schwankungen des Ethen-haltigen Einsatzgemisches nicht mit den Schwankungen des n-Buten-haltigen Einsatzgemisches überlagern. Grundsätzlich verändert sich die Zusammensetzung von $C_2$-Strömen nicht so stark wie die von $C_4$-Strömen, da lediglich ein Ethen-Isomer existiert, während das n-Buten in drei isomeren Strukturen vorliegt.

Vorzugsweise wird das gezielt eingesetzte Ethen aus einer kontinuierlichen Ethen-Quelle bezogen, welche eine Kombination der folgenden Substanzmassenströme liefert, die sich innerhalb der angegebenen Substanzmassenstrombereiche mit einer jeweiligen, im angegebenen Veränderungsgeschwindigkeitsbereich liegenden Veränderungsgeschwindigkeit verändern:

| Substanz | Substanzmassenstrom | Veränd eru ngsgeschwind ig keit |
|---|---|---|
| Ethen: | 0.1 kg/s bis 5 kg/s | - 0.01 $g/s^2$ bis 0.01 $g/s^2$ |
| n-Buten: | 0 kg/s bis 0.01 kg/s | - 0.01 $g/s^2$ bis 0.01 $g/s^2$ |
| Sonstige Stoffe: | 0 kg/s bis 1 kg/s | - 0.05 $g/s^2$ bis 0.05 $g/s^2$ |

Im Vergleich zu der Spezifikation des $C_4$-Stroms ist der $C_2$-Strom weitestgehend konstant. Der guten Ordnung halber wurde klargestellt, dass der $C_2$-Strom praktisch kein n-Buten enthält.

Alle die hier beschriebenen Olefin-Oligomerisierungen werden vorzugsweise in der Flüssigphase in Gegenwart eines festen, heterogenen Katalysators durchgeführt, welcher Nickel, Silicium und Aluminium enthält. Offenbart ist ein geeigneter Katalysator in US2581228.

Die Hydroformylierung erfolgt hingegen in Gegenwart eines homogenen Katalysatorsystems, welches entweder Kobalt oder Rhodium umfasst. Rh basierte Katalysatorsysteme können zusätzlich eine Organophosphorverbindung als Ligand umfassen. Geeignete Katalysatorsysteme besprechen Franke et al. in ihrem oben zitierten Aufsatz.

Der Austrag der Hydroformylierung wird einer Hydrierung unterzogen, um die enthaltenen Aldehyde zu Alkoholen umzuwandeln. Die katalytische Hydrierung von Aldehyden zu Alkoholen ist in der Literatur breit beschrieben, sie kann sowohl heterogen, wie auch homogen katalysiert ablaufen. Einen Überblick liefern:

J. Falbe, H. Bahrmann, W. Lipps, D. Mayer, G. D. Frey, Alcohols Aliphatic in Ullmann's Encyclopedia of Industrial Chemistry, 2013
und

D. Sanfilippo, P. N. Rylander, Hydrogenation and Dehydrogenation in Ullmann's Encyclopedia of Industrial Chemistry, 2009.

Der Hydrierprozess hat keinen messbaren Einfluss auf die Isomerenverteilung, d.h. die Skelettisomeren des Aldehyds finden sich in gleichem Verhältnis im Alkohol wieder.

Die Umsetzung des Alkoholgemisches in das Estergemisch erfolgt vorzugsweise dadurch, dass das Alkoholgemisch mit einer Carbonsäure oder einem Carbonsäureanhydrid verestert wird. Dies sind an sich bekannte Verfahren, deren Details EP1186593B1 und EP1300388B1 entnommen werden können. Das Estergemisch kann prinzipiell auch durch Umesterung eines bestehenden Esters mit dem $C_9$-Alkohol hergestellt werden. EP1430014B1 zeigt, dass der Prozessschritt der Estererzeugung keinen Einfluss auf die Qualität des Weichmachers hat; diese wird ausschließlich von den Eigenschaften des $C_9$-Alkohols bestimmt.

Die von dem Verfahren umfassten Reaktionsschritte Oligomerisierung, Hydroformylierung und Hydrierung werden vorzugsweise kontinuierlich durchgeführt, da die eingesetzten $C_4$-Quellen auch kontinuierlich Rohstoff liefern. Einzig der Veresterungsschritt erfolgt vorzugsweise im Batch-Betrieb, da es sich hierbei um eine Gleichgewichtsreaktion handelt, deren Gleichgewicht über die Destillation in Richtung des Esters verschoben werden muss.

Sofern es sich bei dem erhaltenen Alkoholgemisch um ein Gemisch aus isomeren Nonylalkoholen (INA) wie zum Beispiel n-Nonanol und einfach und/oder mehrfach verzweigten Nonanolen wie insbesondere Methyloctanol handelt, bietet es sich an, dieses mit Phthalsäure oder Phthalsäureanhydrid zu Diisononylphthalat (DINP) umzusetzen. Bei dem DINP handelt es sich dann um ein Estergemisch, welches sich als Weichmacher für PVC einsetzen lässt.

Der Ablauf von verschiedenen Verfahrensvarianten soll nun anhand von vereinfachten Fließbildern erläutert werden. Hierfür zeigen:

Figur 1: Erste Ausführungsform des erfindungsgemäßen Verfahrens mit separater Ethen- und Buten-Oligomerisierung

Figur 2: Weiterbildung der ersten Ausführungsform des erfindungsgemäßen Verfahrens, bei der das Ethen in Gegenwart von Hexen oligomerisiert wird

Figur 3: Erste Variante der in Figur 2 gezeigten Ausführungsform, bei der das Hexen durch Trimerisierung von Ethen erhalten wird

Figur 4: Zweite Variante der in Figur 2 gezeigten Ausführungsform, bei der das Hexen durch Dimerisierung von Propen erhalten wird

Figur 5: Zweite Ausführungsform des erfindungsgemäßen Verfahrens mit Co-Oligomerisierung von Ethen und Buten

Figur 6: Erste Ausführungsform des erfindungsgemäßen Verfahrens mit separater Ethen- und Buten-Oligomerisierung, wobei die Ethen-Oligomerisierung in Gegenwart eines inerten Lösungsmittels erfolgt

Figur 7: Dritte Variante der in Figur 2 gezeigten Ausführungsform, bei der das Hexen als Nebenprodukt der Ethen-Oligomerisierung erhalten wird

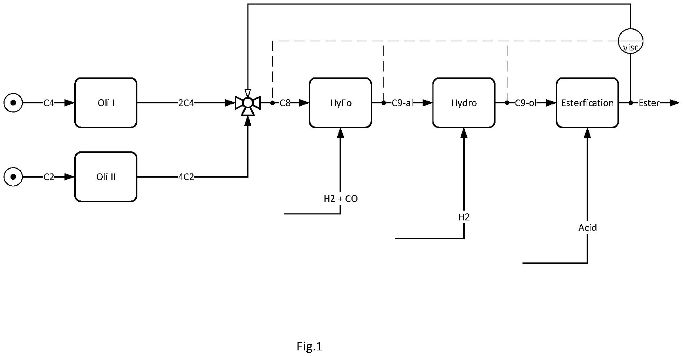

In der in Figur 1 gezeigten ersten Ausführungsform des Verfahrens wird in bekannter Weise ein Einsatzgemisch enthaltend n-Buten (C4) einer ersten Oligomerisierung (Oli I) unterworfen. Dabei entsteht ein Oligomerisat, welches verschiedene Buten-Oligomere, unter anderem Dimere, Trimere und Tetratmere des n-Butens, enthält. Für das Verfahren von Interesse sind lediglich die Dimere des n-Butens, das Dibuten (2C4), welches ein Gemisch verschiedener $C_8$-Olefine darstellt. Das Dibuten (2C4) wird aus dem Oligomerisat der ersten Oligomerisierung (Oli I) destillativ abgetrennt, was in der Figur aus Gründen der Vereinfachung nicht dargestellt ist.

Parallel wird ein Ethen (C2) enthaltendes Einsatzgemisch einer zweiten Oligomerisierung (Oli II) unterworfen. Die Ethen-Oligomerisierung (Oli II) und die Buten-Oligomerisierung (Oli I) laufen in unterschiedlichen Reaktoren, also räumlich getrennt voneinander, ab.

In der zweiten Oligomerisierung (Oli II) entstehen verschiedene Ethen-Oligomere, unter anderem die Tetramere des Ethens (4C2), die wiederum ein Gemisch verschiedener Olefine mit acht Kohlenstoffatomen (C8) darstellen.

Von dem Dibuten (2C4) unterscheiden sich die Ethen-Tetramere (4C2) durch ihren geringeren Verzweigungsgrad.

Die Ethen- und Buten-Oligomere werden zu einem Gemisch von Olefinen mit acht Kohlenstoffatomen (C8) vermengt. Dabei entsteht ein physikalisches Gemisch der beiden Oligomer-Arten. Eine chemische Reaktion zwischen den $C_8$-Olefinen zu höheren Oligomeren findet aufgrund des fehlenden Katalysators nicht statt. Die Zugabe der Ethen-Oligomere zu den Buten-Oligomeren erfolgt gezielt in Abhängigkeit einer Näherung der Ist-Viskosität des Verfahrensprodukts, des Estergemisches (Ester). Zu diesem Zweck wird die Näherung der Ist-Viskosität des Estergemisches von einem Instrument (visc) bestimmt. Die Bestimmung erfolgt durch Messung und/oder durch rechnerische Vorhersage aus der Analytik der Vorprodukte Olefingemisch (C8), Aldehydgemisch (C9-al) oder Alkoholgemisch (C9-ol). In den Zeichnungen ist die rechnerische Bestimmung gestrichelt dargestellt, die Messung mit einer durchgezogenen Linie zum Instrument (visc).

Da ein hoher Anteil an Ethen-Oligomeren (4C2) in dem zu oxierenden Gemisch von Olefinen mit acht Kohlenstoffatomen (C8) die Viskosität des Estergemisches senkt, wird der Anteil an Ethen-Oligomeren in dem zu oxierenden Olefingemisch (C8) gezielt erhöht, wenn die gegenwärtig bestimmte Näherung der Ist-Viskosität eine zuvor festgelegte Soll-Viskosität übersteigt. Umgekehrt wird der Anteil an Ethen-Oligomeren in dem zu oxierenden Olefingemisch (C8) gezielt reduziert, wenn ein Vergleich der Näherung der Ist-Viskosität mit der Soll-Viskosität ergibt, dass die Ist-Viskosität zu gering ist.

Das Einstellen des Mischungsverhältnisses der Ethen-Tetramere (4C2) zu den Buten-Dimeren (2C4) innerhalb des zu oxierenden Olefingemisches C8 erfolgt im einfachsten Fall manuell durch den Anlagenfahrer und idealerweise automatisiert durch übliche Regelungstechnik.

Die übrigen Verfahrensschritte zur Herstellung des Estergemisches (Ester) entsprechen dem anhand von Figur 0 geschilderten Stand der Technik.

Die Tatsache, dass stromabwärts nach der Vermischung der Ethen- und Buten-Oligomere der Verfahrensablauf dem Stand der Technik entspricht, zeigt, dass eine bestehende Anlage zur Herstellung von Estergemischen auf Basis von n-Buten mit überschaubaren Aufwand zur Durchführung des Erfindungsgemäßen Verfahrens aufgerüstet werden kann, nämlich durch Installation der C2-Oligomerisierung (Oli II) und der Apparatur zum Vermischen der Ethen- und Buten-Oligomere nebst dessen Steuerung in Abhängigkeit der Näherung der Ist-Viskosität.

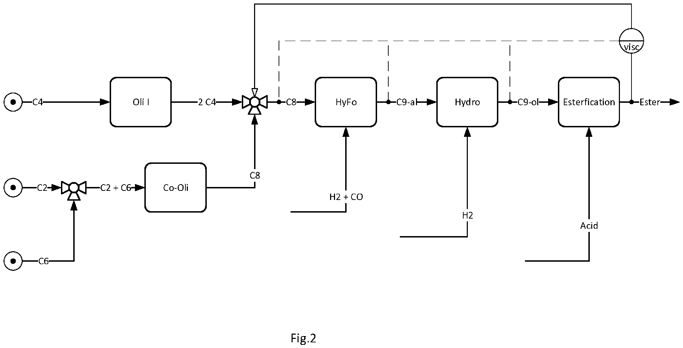

Figur 2 zeigt das Fließbild einer bevorzugten Weiterbildung der in Figur 1 gezeigten Ausführungsform. Diese ist dadurch gekennzeichnet, dass das Ethen (C2) in Gegenwart von Olefinen mit sechs Kohlenstoffatomen (C6) oligomerisiert wird. Die zweite Oligomerisierung ist mithin eine Co-Oligomerisierung (Co-Oli) von Ethen und Hexen. Die Co-Oligomerisierung von Ethen und Hexen hat den Vorteil, dass das Hexen als Lösemittel für das Ethen verwendet werden kann, sodass die Co-Oligomerisierung in der flüssigen Phase an einem festen Katalysator erfolgen kann (heterogene Katalyse). Dies erleichtert die Wärmeabfuhr der exothermen Reaktion über das Lösungsmittel, steigert die Prozessintensität und erlaubt die Verwendung des Equipments, welches auch für die $C_4$-Oligomerisierung (Oli I) verwendet wird. Dadurch werden die Entwicklungskosten gesenkt. Die aus der Co-Oligomerisierung von Ethen und Hexen erhaltenen $C_8$-Olefine werden abgetrennt und sodann - wie anhand von Figur 1 erläutert - dem Dibuten (2C4) gezielt zugegeben.

Olefine mit sechs Kohlenstoffatomen (Hexen) sind kein Standard-Rohstoff der Petro-Folgechemie. Die Hexene lassen sich aber durch Oligomerisierung der Standard-Rohstoffe Ethen und Propen herstellen.

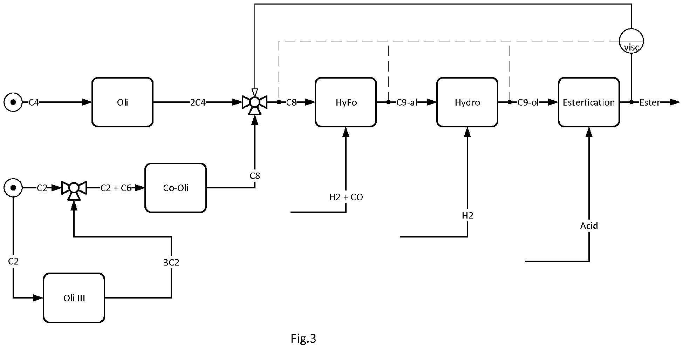

In Figur 3 ist gezeigt, wie das Hexen (C6) durch Trimerisierung des Ethens (C2) gewonnen werden kann. Dafür wird ein Teil des ohnehin erforderlichen Ethens einer von der ersten und zweiten Oligomerisierung separat durchgeführten dritten Oligomerisierung (Oli III) unterworfen und die dabei erhaltenen Ethen-Trimere (3C2) werden abgetrennt.

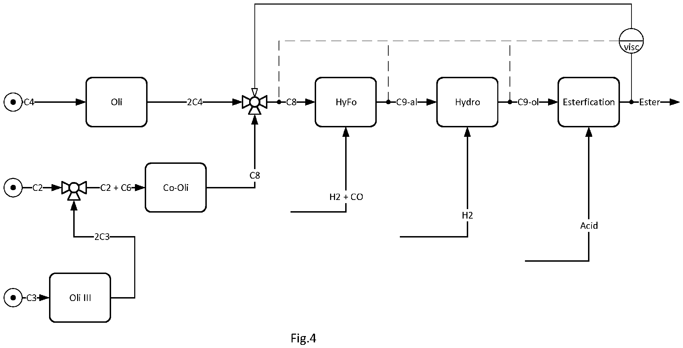

Sofern neben der $C_4$-Quelle und $C_2$-Quelle noch eine $C_3$-Quelle verfügbar ist, kann das Hexen auch dadurch gewonnen werden, dass Propen (C3) einer von der ersten und zweiten Oligomerisierung separat durchgeführten dritten Oligomerisierung (Oli III) unterworfen wird. Die dabei entstehenden Propen-Dimere (2C3) werden abgetrennt und mit dem Ethen Co-oligomerisiert. Diese Variante ist in Figur 4 gezeigt.

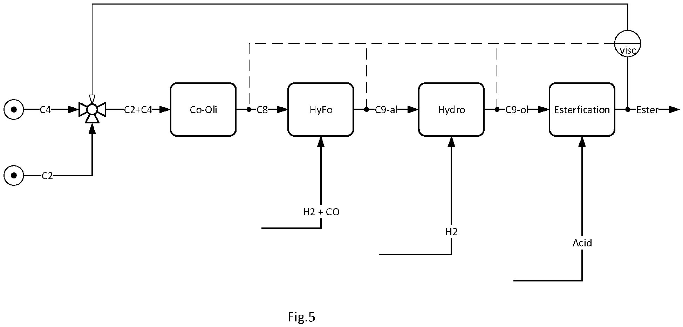

Die zweite Ausführungsform der Erfindung sieht vor, dass das Ethen mit dem Buten gemeinsam einer Co-Oligomerisierung unterworfen wird, anstatt die separat erzeugten Oligomere zu vermischen. Die Vermischung findet demnach vor der Oligomerisierung statt und nicht danach. Ein entsprechendes Verfahrensfließbild ist in Figur 5 dargestellt.

Demnach wird in Abhängigkeit von der Näherung der Ist-Viskosität des Estergemisches dem n-Buten enthaltenden Einsatzgemisch (C4) Ethen (C2) aus einer konstanten C2-Quelle zugegeben. Sofern die Näherung der Ist-Viskosität geringer ist als die zuvor festgelegte Soll-Viskosität, wird die Ethen-Zugabe gedrosselt. Ergibt der Vergleich, dass die Näherung der Ist-Viskosität größer ist als die Soll-Viskosität, wird dem n-Buten-haltigen Einsatzgemisch mehr Ethen zugemischt.

Das Gemisch enthaltend n-Buten und Ethen (C2+C4) wird sodann einer Co-Oligomerisierung (Co-Oli) unterworfen, durchgeführt in einem gemeinsamen Reaktionsgefäß.

Aus dem Oligomerisat der Co-Oligomerisierung (Co-Oli) werden die Olefine mit acht Kohlenstoffatomen (C8) destillativ abgetrennt, was in der Zeichnung nicht dargestellt ist. Die C8-Olefine dienen dann als Einsatzmaterial für die Hydroformylierung (Hydro). Die Hydroformylierung und die übrigen Prozessschritte erfolgen wie in der ersten Ausführungsform.

Die Tatsache, dass stromabwärts nach der Vermischung des Ethens und des Butens das Equipment dem Stand der Technik entspricht, zeigt, dass eine bestehende Anlage zur Herstellung von Estergemischen auf Basis von n-Buten mit überschaubaren Aufwand zur Durchführung des Verfahrens aufgerüstet werden kann, nämlich durch Umbau der $C_4$-Oligomerisierung in eine C2/C4-Co-Oligomerisierung und durch Installation der Apparatur zum Zugeben des Ethens in das n-Buten-haltige Einsatzgemisch nebst dessen Steuerung in Abhängigkeit der Näherung der Ist-Viskosität.

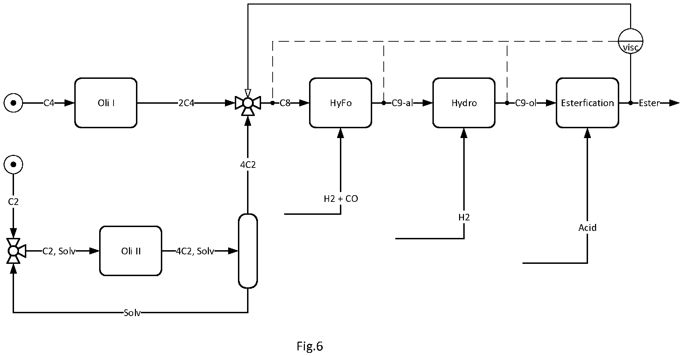

Figur 6 zeigt eine spezielle Ausgestaltung des Verfahrens mit getrennter Buten- und Ethen-Oligomerisierung: Um die zweite Oligomerisierung (Oli II) in der flüssigen Phase durchführen zu können, wird das gasförmig aus der Quelle bezogene Ethen (C2) zunächst in einem inerten, flüssigen Lösungsmittel (Solv) gelöst. Als Lösungsmittel eignen sich vor allem Alkane wie Propan, Isobutan, Pentan, Hexan, Heptan. Die Ethen-Oligomerisierung (Oli II) erfolgt dann innerhalb des flüssigen Lösungsmittels an dem festen heterogenen Katalysator. Die dabei erhaltenen Ethen-Tetramere liegen dann in dem Lösungsmittel gelöst vor (4C2, Solv). Bevor die Ethen-Tetramere (4C2) mit dem Dibuten (2C4) vermischt werden, muss das Lösungsmittel (solv) abgetrennt werden. Dies erfolgt in einer hier ausnahmsweise dargestellten Destillationskolonne. Der Siedepunkt bzw. Siedebereich des Lösungsmittels sollte von den Ethen-Oligomeren möglichst weit entfernt sein, um die Trennung zu vereinfachen. Liegt er oberhalb der Ethen-Tetramere, werden diese wie in Figur 6 dargestellt über Kopf abgetrennt. Das hochsiedende Lösungsmittel verbleibt im Sumpf der Destillation, wird rezykliert und mit frischem Ethen von der $C_2$-Quelle vermischt. Der übrige Prozess entspricht dem aus Figur 1 bekannten.

Eine weitere Ausführungsform des Verfahrens ist dadurch gekennzeichnet, dass das Lösungsmittel Hexen als Nebenprodukt der Ethen-Oligomerisierung erhalten wird.

Hintergrund davon ist, dass auch bei der Co-Oligomerisierung von Ethen und Hexen neben $C_8$ auch wiederum neues $C_6$-Olefin gebildet wird. Über geeignete Reaktionsbedingungen und ggf. durch Anwesenheit eines weiteren, inerten Lösungsmittels lässt sich in der Bilanz die Menge an gebildetem zum verbrauchten $C_6$-Olefin soweit konstant halten, so dass ein konstanter Kreislauf möglich ist. Alternativ würde man neben den $C_8$-Olefinen bei jeder Ethen-Oligomerisierung auch größere Mengen an Hexen als Nebenprodukt abtrennen und anderweitig verwenden.

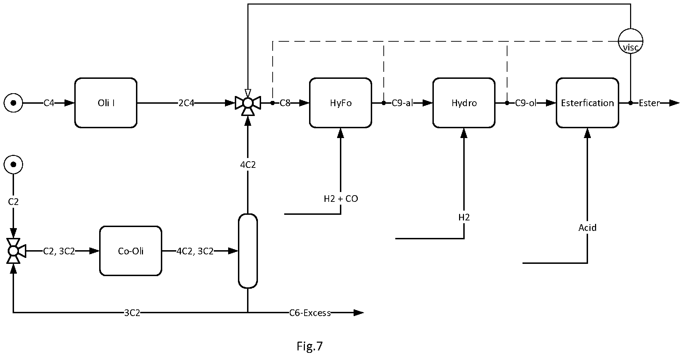

Ein entsprechendes Verfahrensfließbild zeigt Figur 7. Der apparative Aufbau entspricht im Wesentlichen dem in Figur 2 gezeigten. Als Lösungsmittel wird aber nicht ausschließlich ein inertes Lösungsmittel verwendet, sondern reaktives Hexen. Bei dem Hexen handelt es sich um Trimere des Ethens (3C2), die in der Co-Oligomerisierung (Co-Oli) von Ethen und Hexen gebildet werden. Die als Lösungsmittel dienenden Ethen-Trimere (3C2) werden aus dem Oligomerisat der Co-Oligomerisierung (Co-Oli) destillativ abgetrennt, zumindest teilweise rezykliert und mit frischem Ethen vermischt. Sofern vorhanden, wird überschüssiges Hexen (C6-Excess) ausgeschleust und anderweitig verwendet. Neben Hexen kann in diesem Prozess auch ein inertes Lösungsmittel parallel verwendet werden, was ebenfalls im Kreislauf geführt wird.

Die folgenden experimentellen Ergebnissen belegen, wie sich die Viskosität von auf n-Buten basierenden Estergemischen durch gezielten Einsatz von Ethen bei der Herstellung der Ester-Vorprodukte beeinflussen lässt:

### Beispiel 1: Oligomerisierung von Butenen zu Di-n-Buten

355 g eines heterogenen Katalysators basierend auf Nickel und Silica-Alumina (vgl. US 2581228) wurden in einen außen mit Öl temperierten, weitestgehend isotherm betriebenen Rohrreaktor von 2 m Länge und 2.1 cm Innendurchmesser gefüllt. Als Wärmeträger diente das Produkt Marlotherm der Firma Sasol. Die Reaktion wurde bei einem Absolutdruck von $30*10^5$ Pa in der Flüssigphase durchgeführt. Anschließend wurde darüber in geradem Durchgang ein Raffinat III genannter Einsatzstoff, dessen Zusammensetzung in Tabelle 1 abgebildet ist, mit einem Mengenstrom von 1 kg/h bei einer Rohrmanteltemperatur von 80 °C gefahren und im Zuge dessen oligomerisiert. Nach einer Zeit von 40 h war ein Zustand erreicht, in dem sich der Umsatz nicht mehr änderte.

Tabelle 1: Zusammensetzung von Raffinat III

| 1-Buten | *cis*-2-Buten | *trans*-2-Buten | *n*-Butan | Isobutan | Sonstige Stoffe |
|---|---|---|---|---|---|
| [Gew.-%] | [Gew.-%] | [Gew.-%] | [Gew.-%] | [Gew.-%] | [Gew.-%] |
| 24 bis 29 | 11 bis 16 | 20 bis 24 | 16-18 | 21-23 | weniger als 0.5 |

Hinter der Reaktionsstufe wurden die Oligomere von den Butanen und nicht umgesetzten Butenen abgetrennt und auf ihre Zusammensetzung analysiert. Zur Identifizierung der Octen-Skelettisomere wurde eine hydrierende GC-Analytik verwendet, bei der die oligomeren Olefine zunächst zu Alkanen hydriert werden. Die so erhaltenen Alkane wurden dann gaschromatographisch getrennt und detektiert. Man kann zwischen drei relevanten $C_8$-Isomeren differenzieren: lineares C8 (gebildet aus n-Octenen), einfach verzweigtes C8 (gebildet aus Methylheptenen) und zweifach verzweigtes C8 (gebildet aus Dimethylhexenen). Die Ergebnisse sind in Tabelle 2 zusammengefasst.

Um den betrachteten Gesamtprozess abzubilden, wurde der Produktaustrag gesammelt (ca. 5 kg) und einer destillativen Trennung unterzogen, um die gewünschte Cs-Fraktion von den verbliebenen leichter siedenden nicht umgesetzten Butenen und Butanen und den höher siedenden schwereren Oligomeren und Nebenprodukten zu trennen. Das gereinigte Produkt wurde für die Hydroformylierung bereitgestellt.

### Beispiel 2: Oligomerisierung von Ethen im Lösungsmittel Isobutan

263 g eines heterogenen Katalysators basierend auf Nickel und Silica-Alumina (vgl. US 2581228) wurden in einen außen mit Öl temperierten, mit einem Thermoelement versehenen Rohrreaktor von 2 m Länge und 2.1 cm Innendurchmesser gefüllt. Anschließend wurde darüber in geradem Durchgang ein Gemisch aus durchschnittlich 13 Gew.-% Ethen und 87 Gew.-% Isobutan mit einem Gesamtmengenstrom von 1 kg/h bei einer Vorgabetemperatur von 30 °C gefahren. Der Druck wurde auf $30*10^5$ Pa konstant gehalten. Nach einer Zeit von 106 h war ein Zustand erreicht, in dem sich der Umsatz nicht mehr änderte. Die Ergebnisse der Ethenoligomerisierung sind ebenfalls in Tabelle 2 zusammengefasst. Das Produktgemisch wurde erneut gesammelt und das entstandene C8 durch Destillation von höher und leichter siedenden Verunreinigungen befreit und für die Hydroformylierung bereitgestellt.

**Beispiel 3: Co-Oligomerisierung von Ethen und 1-Hexen im Lösungsmittel n-Hexan**

501 g eines heterogenen Katalysators basierend auf Nickel und Silica-Alumina (vgl. US 2581228) wurden in einen außen mit Öl temperierten, mit einem Thermoelement versehenen Rohrreaktor von 2 m Länge und 2.1 cm Innendurchmesser gefüllt. Anschließend wurde darüber in geradem Durchgang ein Gemisch aus durchschnittlich 10 Gew.-% Ethen, 49 Gew.-% 1-Hexen und 41 Gew.-% n-Hexan mit einem Gesamtmengenstrom von 1 kg/h bei einer Vorgabetemperatur von 50 °C gefahren. Der Druck wurde auf $30*10^5$ Pa konstant gehalten. Die Ergebnisse der Co-Oligomerisierung von Ethen und 1-Hexen sind ebenfalls in Tabelle 2 zusammengefasst. Das Produktgemisch wurde erneut gesammelt und das entstandene C8 durch Destillation von höher und leichter siedenden Verunreinigungen befreit und für die Hydroformylierung bereitgestellt.

Tabelle 2: Ergebnisse der Oligomerisierungsversuche

| Bsp. | Feed | WHSV | X(C4) | X(C2) | Anteil C8 | C8-Sel. | | |
|---|---|---|---|---|---|---|---|---|
| | | | | | | zweifach verzweigt | einfach verzweigt | linear |
| 1 | C4 | 2,8 $h^{-1}$ | 43% | - | 86% | 25% | 59% | 16% |
| 2 | C2 | 4,7 $h^{-1}$ | - | 99% | 7% | 11% | 48% | 39% |
| 3 | C2 + C6 | 2,0 $h^{-1}$ | - | 87% | 89% | 1% | 25% | 73% |

**Beispiel 4: Hydroformylierung der verschiedenen $C_8$-Olefingemische**

Es wurden jeweils 0.013 g $Rh(acac)(CO)_2$ und 0.67 g Tris(2,4-ditert-butylphenyl)phosphit in ein mit Argon inertisiertes 100 mL Schlenkgefäß eingewogen. Anschließend wurden im Argon-Gegenstrom 50 g $C_8$-Olefin in das Schlenkgefäß überführt. Die entstandene Lösung wurde dann in einen ebenfalls mit Argon inertisierten 100 mL-Autoklaven der Fa. Parr Instruments überführt. Der Autoklav war mit einem Gaseintragsrührer ausgestattet. Beim Versuchsstart wurde der Rührer mit 1200 u/min in Betrieb genommen und mit $20*10^5$ Pa Synthesegas (CO/H2 = 1:1 (Vol-%)) beaufschlagt. Anschließend wurde der Autoklav auf 120 °C temperiert. Nach Erreichen der Reaktionstemperatur wurde der Synthesegasdruck auf $50*10^5$ Pa erhöht. Während der Reaktion wurde der Druck über eine Druckregelung mit Synthesegas konstant gehalten. Die Reaktion wurde für 17 h betrieben. Es wurde jeweils bis zum Vollumsatz hydroformyliert. Der Reaktionsaustrag wurde nach Abkühlen und Entspannen des Autoklaven in ein 1 L großes mit Argon gespültes Schlenkgefäß überführt.

Dieser Versuch wurde für jedes Olefin insgesamt fünfmal wiederholt und die fünf Reaktionsausträge der einzelnen Versuche für jeweils ein Olefin wurden vereinigt. Die drei Produktfraktionen der gesammelten Reaktionsausträge wurden dann mittels Destillation mit Hilfe eines Rotationsverdampfers der Fa. Heidoplh (Typ Laborota 4002 mit ILMVAC-Pumpe) bei 1 mbar und 120 °C bei 100 u/min vom Katalysator abgetrennt, wobei die Produkte, Gemische aus $C_9$-Aldehyden, über Kopf genommen wurden. Die Ausbeuten an $C_9$-Aldehyd betrugen jeweils etwa 96%.

**Beispiel 5: Hydrierung der verschiedenen $C_9$-Aldehyd-Gemische zu den entsprechenden $C_9$-Alkohol-Gemischen**

180 g (1.26 mol) des jeweiligen Aldehyd-Gemischs aus der Hydroformylierung wurden mit 150 mL Methanol verdünnt und auf 0 °C abgekühlt. Zu dieser Lösung wurde langsam unter Rühren entsprechend der Masse des Aldehyds 2 Äquivalente Natriumborhydrid (23.9 g, 0.63 mol, Sigma Aldrich) zugegeben (entspricht 1 mol-Äquivalent $H_2$). Nach erfolgter Zugabe wurde das Reaktionsgemisch 2 h bei Raumtemperatur gerührt. Die Produktlösung wurde sodann dreimal mit jeweils 200 mL Dichlormethan extrahiert und das produkthaltige Dichlormethan nochmals mit einer gesättigten Ammoniumchlorid-Lösung gewaschen. Anschließend wurde über Natriumsulfat getrocknet und das Dichlormethan von dem $C_9$-Alkohol-Gemisch abdestilliert. Die Produktausbeute betrug jeweils etwa 89%.

**Beispiel 6: Veresterung der $C_9$-Alkohole mit Phthalsäureanhydrid zum Diisononylphthalat (DINP) und Bestimmung der Viskositäten**

In einer Apparatur umfassend 250 mL Destillationskolben mit Magnetrührstäbchen, Probennahmestutzen, Wasserabscheider mit aufgesetztem Intensivkühler sowie Thermometer wurden jeweils 37.02 g (0.25 mol) Phthalsäureanhydrid (Sigma Aldrich), 90 mg (0,1 mol-% bezogen auf Phthalsäureanhydrid) Tetrabutyl-orthotitanat und 79.34 g (0.55 mol) eines nach Beispiel 1 bis 5 hergestellten $C_9$-Alkoholgemisches vorgelegt und bei 220 °C verestert. Zur schnellen Entfernung von Wasser wurden 40 g Cyclohexan als Schleppmittel hinzugegeben. Nach 7 Stunden war die Reaktion beendet. Der überschüssige Alkohol sowie das Schleppmittel wurden bei 180 °C und einem Vakuum von 3 mbar abdestilliert. Anschließend wurde auf 80 °C abgekühlt, mit 1 mL einer 10 Gew.-%igen wässrigen NaOH-Lösung

in 10 mL $H_2O$ neutralisiert und 15 Minuten bei Normaldruck gerührt. Danach wurde der Ansatz bei dieser Temperatur bei 500 Pa getrocknet und im Anschluss bei einer Temperatur von 180 °C und einem Druck zwischen 500 Pa und 100 Pa durch Einleiten von Stickstoff von Spuren an Wasser und Alkohol befreit. Nach Abkühlen auf 80 °C wurde das Produkt filtriert. Die Reinheit des Produkts wurde mittels GC-Chromatographie bestimmt. Die Viskosität der Produkte wurde mit einem Stabinger Viscometer TM SVMTM 3000 (Anton Paar) in Anlehnung an ASTM D 7042 gemessen.

**Viskositätsmessung gemäß ASTM D7042**

Das Stabinger Viskosimeter SVM 3000 ist ein Kombigerät, mit dem Dichte und Viskosität bestimmt werden können. Dazu sind in dem Gerät zwei Messzellen hinter einander geschaltet. Zur Bestimmung der Viskosität ist ein Rotationsviskosimeter mit Zylindergeometrie verbaut und zur Bestimmung der Dichte eine Dichtemesszelle nach dem Biegeschwingerprinzip. Durch einmaliges Einspritzen der Probe werden so beide Messwerte bestimmt. Gemessen werden die Proben bei 20 °C. Temperiert werden die Messzellen über ein Peltier Element (Reproduzierbarkeit 0,02 °C). Gemessen werden die Proben mit dem Voreingestellten Messmodus "M0-ASTM (PRECISE)" Messung mit höchster Genauigkeit und Wiederholungen, für Tests nach der Norm ASTM D7042. Dazu wird für jede Messung ca. 0,5 ml Probe nachdosiert (um Lufteinschlüsse oder Verunreinigungen auszuschließen). Bei den internen Wiederholungen wird erst dann ein gültiges Ergebnis angezeigt, wenn die Abweichung der Werte nicht höher als +/-0,1% des Messwerts für die Viskosität und +/-0,0002 g/cm$^3$ für die Dichte ist. Zusätzlich zu den internen Wiederholungen wird von jeder Probe eine Doppelbestimmung durchgeführt. Nach jeder Bestimmung wird das Gerät mit Aceton gereinigt und mit Luft getrocknet (eingebaute Pumpe).

Die Ergebnisse der Viskositätsmessungen sind in Tabelle 4 dargestellt.

Tabelle 4: Ergebnisse der dynamischen Viskositätsmessungen

| **C8-Quelle** (C8-Synthese aus Bsp.-No.) | **Viskositäten ausgehend von den Gemischen der C8-Quellen [mPa•s]** | | | | |
|---|---|---|---|---|---|
| | 1:0 | 2:1 | 1:1 | 1:2 | 0:1 |
| 2C4 + 4C2 (1+2) | 97,5 | 91,6 | 88,8 | 86,2 | 81,5 |
| 2C4 + C8 aus C2+C6 (1+3) | 97,5 | 90,3 | 87,1 | 84,1 | 78,7 |

**Beispiel 7: Co-Oligomerisierung von Butenen und Ethen**

Die Experimente zur Oligomerisierung des n-Buten enthaltenden Einsatzstoffes Raffinat III im direkten Vergleich mit der Co-Oligomerisierung von Ethen mit Raffinat III wurden im Labormaßstab in kontinuierlich betriebenen Festbettrohrreaktoren durchgeführt. Hierzu wurden 12 g eines heterogenen Katalysators basierend auf Nickel und Silica-Alumina (vgl. US 2581228) in einen außen mit Öl temperierten Rohrreaktor von 1,6 m Länge und 0,7 cm Innendurchmesser gefüllt. Anschließend wurden darüber in geradem Durchgang zum einen Raffinat III, zum anderen ein Gemisch aus 2 Gew.-% Ethen und 98 Gew.-% Raffinat III mit einem Gesamtmengenstrom zwischen 75 und 184 g/h bei einer Vorgabetemperatur von 80 °C gefahren. Die Reaktion wurde bei einem Absolutdruck von $28*10^5$ Pa in der Flüssigphase durchgeführt. Die Zusammensetzung des Raffinat III ist in Tabelle 1 abgebildet. Die Analytik des Reaktoreintrags und -austrags erfolgte mittels online GC-Analytik. Durch Variation der spezifischen Raumbelastung (weight hourly space velocity WHSV) zwischen 1,5 $h^{-1}$ und 25 $h^{-1}$ wurden verschiedene vergleichbare Zielumsätze eingestellt.

Die erhaltene $C_8$-Produktselektivität sowie die Selektivitäten bezüglich linearer, einfach und zweifach verzweigter Octene sind in Tabelle 5 beispielhaft für zwei verschiedene Versuchspaare mit jeweils vergleichbaren Butenumsätzen wiedergegeben. Die Butenumsätze X(C4) wurden aus der Differenz des Butenanteils im Reaktoreintrag und des Butenanteils im Reaktoraustrag berechnet (Gleichung 1). Die im Raffinateingangsstrom enthaltenen Butene und die intermediär durch die Dimerisierung des Ethens gebildeten Butene wurden hierbei nicht weiter differenziert betrachtet. Ethen wurde in den hier aufgeführten Beispielen zu 100 % umgesetzt.

$$X_{C4} = (m'_{Butene,0} - m'_{Butene,i}) / m'_{Butene,0} \quad \text{(Gleichung 1)}$$

Tabelle 5: Ergebnisse der Oligomerisierung von Raffinat III im direkten Vergleich zur Co-Oligomerisierung von Raffinat III und Ethen.

| No. | Feed | WHSV | X(C4) | X(C2) | Anteil C8 | C8-Sel. zweifach verzweigt | C8-Sel. einfach verzweigt | C8-Sel. linear |
|---|---|---|---|---|---|---|---|---|
| 6a | C4 | 12,5 $h^{-1}$ | 10% | - | 92% | 13% | 57% | 28% |
| 6b | C4+C2 | 15,3 $h^{-1}$ | 9% | 100% | 50% | 12% | 56% | 31% |
| 6c | C4 | 6,3 $h^{-1}$ | 14% | - | 91% | 15% | 58% | 24% |
| 6d | C4+C2 | 9,2 $h^{-1}$ | 13% | 100% | 56% | 14% | 57% | 27% |

**Fazit:**

Bei dem Vergleich der $C_8$-Isomeren in Tabelle 2 wird schnell deutlich, dass die Oligomerisierung von reinem Ethen sowie die Co-Oligomerisierung von Ethen mit Hexen zu mehr linearen und deutlich weniger zweifach verzweigten $C_8$-Olefinen führen. Zieht man die Ergebnisse der Viskositätsmessungen der daraus synthetisierten veresterten Produkte hinzu, so wird deutlich, dass der höhere Anteil an linearen und der geringere Anteil an zweifach verzweigten $C_8$-Olefinen in Folge der Verwendung von Ethen zu einer Absenkung der Viskositäten der entsprechenden Weichmacher in Abhängigkeit der eingesetzten Mengen an Ethen führen. Dabei lässt sich zwischen der sinkenden Viskosität und dem zunehmenden Anteil an Ethen-basierten Cs-Gemischen ein annähernd linearer Zusammenhang erkennen.

Der direkte Vergleich der reinen Buten-Oligomerisierung mit der Co-Oligomerisierung von 2% Ethen mit 98% Buten zeigt, dass die Selektivität zu linearem $C_8$-Olefin bei jeweils in etwa gleichen Umsätzen in der Co-Oligomerisierung um ca. 2 % höher und die Selektivität zum zweifach verzweigten $C_8$-Olefin zu 1% geringer ist als in der reinen Buten-Oligomerisierung. Gemäß der in den vorherigen Beispielen gezeigten Zusammenhänge zwischen der Linearität der $C_8$-Olefine und der daraus resultierenden Viskosität des späteren Weichmachers ist also auch in der Co-Oligomerisierung von Ethen mit Butenen durch den positiven Effekt des Ethens eine geringere Viskosität des Weichmachers zu erwarten.

**Bezugszeichenliste**

| | |
|---|---|
| C4 | Olefine mit vier Kohlenstoffatomen |
| Oli | Oligomerisierung |
| C8 | Olefine mit acht Kohlenstoffatomen |
| 2C4 | Dibuten |
| HyFo | Hydroformylierung |
| H2 | Wasserstoff |
| CO | Kohlenmonoxid |
| H2+CO | Synthesegas |
| C9-al | Aldehyde mit neun Kohlenstoffatomen |
| Hydro | Hydrierung |
| C9-ol | Alkohole mit neun Kohlenstoffatomen |
| Esterfication | Veresterung |
| Acid | Säure bzw. Anhydrid |
| Ester | Estergemisch |
| Visc | Instrument zur Bestimmung der Viskosität |
| Oli I | erste Oligomerisierung |
| C2 | Ethen |
| Oli II | zweite Oligomerisierung |
| 4C2 | Tetramere des Ethens |
| Co-Oli | Co-Oligomerisierung |
| C6 | Olefine mit sechs Kohlenstoffatomen |
| 3C2 | Trimere des Ethens |
| C3 | Propen |
| 2C3 | Dimere des Propens |
| C4+C2 | Gemisch enthaltend n-Buten und Ethen |

| | |
|---|---|
| Solv | Lösemittel |
| C2, Solv | Ethen, im Lösungsmittel gelöst |
| 4C2, Solv | Tetramere des Ethens, im Lösungsmittel gelöst |
| C2+C6 | Gemisch enthaltend Ethen und n-Hexen |
| 4C2, 3C2 | Tetramere des Ethens, in Trimeren des Ethens gelöst |
| C6-Excess | überschüssig gebildete Olefine mit sechs Kohlenstoffatomen |

**Patentansprüche**

**1.** Verfahren zur Herstellung eines Estergemisches, bei welchem ein n-Buten enthaltendes Einsatzgemisch, dessen Zusammensetzung sich über die Zeit verändert, unter Erhalt eines Oligomerisats einer ersten Oligomerisierung unterworfen und zumindest ein Teil der im Oligomerisat enthaltenden Buten-Oligomere in einer Hydroformylierung in Aldehyde oxiert werden, von denen zumindest ein Teil durch anschließende Hydrierung in ein Alkoholgemisch hydriert wird, welches sodann in das Estergemisch umgesetzt wird, und bei welchem eine Näherung der Ist-Viskosität des Estergemisches bestimmt wird, **dadurch gekennzeichnet,**
**dass** in Abhängigkeit von der bestimmten Näherung der Ist-Viskosität des Estergemisches den zu oxierenden Buten-Oligomeren gezielt Ethen-Oligomere zugegeben werden, welche dadurch erhalten sind, dass ein von n-Buten freies, Ethen enthaltendes Einsatzgemisch einer von der ersten Oligomerisierung separat durchgeführten zweiten Oligomerisierung unterworfen wird.

**2.** Verfahren nach Anspruch 1, **dadurch gekennzeichnet, dass** die in Abhängigkeit von der bestimmten Näherung der Ist-Viskosität des Estergemisches den zu oxierenden Buten-Oligomeren gezielt zugegebenen Ethen-Oligomere dadurch erhalten sind, dass ein von n-Buten freies, Ethen und Hexen enthaltendes Einsatzgemisch der von der ersten Oligomerisierung separat durchgeführten zweiten Oligomerisierung unterworfen wird, sodass es sich bei der zweiten Oligomerisierung um eine Co-Oligomerisierung von Ethen und Hexen handelt.

**3.** Verfahren nach Anspruch 2, **dadurch gekennzeichnet,**

a) **dass** das Hexen aus einer von der ersten und zweiten Oligomerisierung separat durchgeführten dritten Oligomerisierung bezogen wird, in welcher Propen dimerisiert wird,
oder
b) **dass** das Hexen aus einer von der ersten und zweiten Oligomerisierung separat durchgeführten dritten Oligomerisierung bezogen wird, in welcher Ethen trimerisiert wird;
oder
c) **dass** das Hexen aus der zweiten Oligomerisierung bezogen wird.

**4.** Verfahren nach Anspruch 1, 2 oder 3, **dadurch gekennzeichnet, dass** die zweite Oligomerisierung in Gegenwart eines flüssigen, inerten Lösungsmittels erfolgt, in welchem das Ethen zumindest teilweise gelöst ist und welches nach Durchführung der zweiten Oligomerisierung von den Ethen-Oligomeren getrennt wird.

**5.** Verfahren nach einem der Ansprüche 1 bis 4, **dadurch gekennzeichnet, dass** die gezielte Zugabe der Ethen-Oligomere zu den zu oxierenden Buten-Oligomeren dergestalt erfolgt, dass zunächst eine Soll-Viskosität des Estergemisches festgelegt wird, dass die bestimmte Näherung der Ist-Viskosität des Estergemisches mit der Soll-Viskosität des Estergemisches verglichen wird, und dass die Menge der den zu oxierenden Buten-Oligomeren zugegebenen Ethen-Oligomere erhöht wird, wenn die Näherung der Ist-Viskosität größer ist als die Soll-Viskosität; bzw. dass die Menge der den zu oxierenden Buten-Oligomeren zugegebenen Ethen-Oligomere verringert wird, wenn die Näherung der Ist-Viskosität kleiner ist als die Soll-Viskosität.

**6.** Verfahren zur Herstellung eines Estergemisches, bei welchem ein n-Buten enthaltendes Einsatzgemisch, dessen Zusammensetzung sich über die Zeit verändert, unter Erhalt eines Oligomerisats einer ersten Oligomerisierung unterworfen und zumindest ein Teil der im Oligomerisat enthaltenden Buten-Oligomere in einer Hydroformylierung in Aldehyde oxiert werden, von denen zumindest ein Teil durch anschließende Hydrierung in ein Alkoholgemisch hydriert wird, welches sodann in das Estergemisch umgesetzt wird, und bei welchem eine Näherung der Ist-Viskosität des Estergemisches bestimmt wird, **dadurch gekennzeichnet,**
**dass** in Abhängigkeit von der bestimmten Näherung der Ist-Viskosität des Estergemisches dem n-Buten enthaltenden Einsatzgemisch gezielt Ethen zugegeben wird, sodass es sich bei der ersten Oligomerisierung um eine Co-Oligomerisierung von Ethen und n-Buten handelt.

7. Verfahren nach Anspruch 6, **dadurch gekennzeichnet, dass** gezielte Zugabe des Ethens zum Einsatzgemisch dergestalt erfolgt, dass zunächst eine Soll-Viskosität des Estergemisches festgelegt wird, dass die bestimmte Näherung der Ist-Viskosität des Estergemisches mit der Soll-Viskosität des Estergemisches verglichen wird, und dass die Menge des dem Einsatzgemisch zugegebenen Ethens erhöht wird, wenn die Näherung der Ist-Viskosität größer ist als die Soll-Viskosität; bzw. dass die Menge des dem Einsatzgemisch zugegebenen Ethens verringert wird, wenn die Näherung der Ist-Viskosität kleiner ist als die Soll-Viskosität.

8. Verfahren nach einem der Ansprüche 1 bis 7, **dadurch gekennzeichnet, dass** die Näherung der Ist-Viskosität des Estergemisches durch Messung der Viskosität des Estergemisches bestimmt wird.

9. Verfahren nach einem der Ansprüche 1 bis 7, **dadurch gekennzeichnet, dass** die Näherung der Ist-Viskosität des Estergemisches durch Berechnung bestimmt wird, wobei die Berechnung wahlweise auf Grundlage einer Analytik des Alkoholgemisches, auf Grundlage einer Analytik der zu hydrierenden Aldehyde oder auf Grundlage einer Analytik der zu oxierenden Oligomere erfolgt.

10. Verfahren nach einem der Ansprüche 1 bis 9,
**dadurch gekennzeichnet,**
**dass** sich das n-Buten-haltige Einsatzgemisch zusammensetzt aus einer Kombination der folgenden Substanzmassenströme, die sich innerhalb der angegebenen Substanzmassenstrombereiche mit einer jeweiligen, im angegebenen Veränderungsgeschwindigkeitsbereich liegenden Veränderungsgeschwindigkeit verändern:

| Substanz | Substanzmassenstrom | Veränderungsgeschwindigkeit |
|---|---|---|
| Isobuten: | 0 kg/s bis 1 kg/s | - 0.05 $g/s^2$ bis 0.05 $g/s^2$ |
| 1-Buten: | 0 kg/s bis 6 kg/s | - 0.30 $g/s^2$ bis 0.30 $g/s^2$ |
| 2-Buten (cis + trans): | 1 kg/s bis 13 kg/s | - 0.30 $g/s^2$ bis 0.30 $g/s^2$ |
| Isobutan: | 0 kg/s bis 3 kg/s | - 0.15 $g/s^2$ bis 0.15 $g/s^2$ |
| n-Butan: | 1 kg/s bis 7 kg/s | - 0.30 $g/s^2$ bis 0.30 $g/s^2$ |
| Sonstige Stoffe: | 0 kg/s bis 1 kg/s | - 0.05 $g/s^2$ bis 0.05 $g/s^2$ |

11. Verfahren nach Anspruch 10,
**dadurch gekennzeichnet,**
**dass** das gezielt eingesetzte Ethen aus einer Ethen-Quelle bezogen wird, welche eine Kombination der folgenden Substanzmassenströme liefert, die sich innerhalb der angegebenen Substanzmassenstrombereiche mit einer jeweiligen, im angegebenen Veränderungsgeschwindigkeitsbereich liegenden Veränderungsgeschwindigkeit verändern:

| Substanz | Substanzmassenstrom | Veränderungsgeschwindigkeit |
|---|---|---|
| Ethen: | 0.1 kg/s bis 5 kg/s | - 0.01 $g/s^2$ bis 0.01 $g/s^2$ |
| n-Buten: | 0 kg/s bis 0.01 kg/s | - 0.01 $g/s^2$ bis 0.01 $g/s^2$ |
| Sonstige Stoffe: | 0 kg/s bis 1 kg/s | - 0.05 $g/s^2$ bis 0.05 $g/s^2$ |

12. Verfahren nach einem der Ansprüche 1 bis 11, **dadurch gekennzeichnet, dass** die erste Oligomerisierung und/oder die zweite und/oder die dritte Oligomerisierung in Gegenwart eines heterogenen Katalysators erfolgt, welcher Nickel, Silicium und Aluminium enthält.

13. Verfahren nach einem der Ansprüche 1 bis 12, **dadurch gekennzeichnet, dass** die Hydroformylierung in Gegenwart eines homogenen Katalysatorsystems erfolgt, welcher entweder Kobalt oder Rhodium umfasst.

14. Verfahren nach einem der Ansprüche 1 bis 13, **dadurch gekennzeichnet, dass** die Umsetzung des Alkoholgemisches in das Estergemisch dadurch erfolgt, dass das Alkoholgemisch mit einer Carbonsäure oder einem Carbonsäureanhydrid verestert wird oder dass das Estergemisch mittels einer Umesterung eines bestehenden Esters mit dem Alkoholgemisch gewonnen wird.

**15.** Verfahren nach Anspruch 14, **dadurch gekennzeichnet, dass** es sich bei dem Alkoholgemisch um ein Gemisch aus isomeren Nonylalkoholen handelt, welches mit Phthalsäure oder Phthalsäureanhydrid zu Diisononylphthalat umgesetzt wird.

## Claims

**1.** Process for preparing an ester mixture in which an n-butene-containing feed mixture having a composition which varies over time is subjected to a first oligomerization to obtain an oligomerizate and at least some of the butene oligomers present in the oligomerizate are hydroformylated to aldehydes, of which at least some are hydrogenated by subsequent hydrogenation to an alcohol mixture which is then converted to the ester mixture, and in which an approximation of the actual viscosity of the ester mixture is determined,
**characterized in that**
as a function of the approximation of the actual viscosity of the ester mixture determined, ethene oligomers which are obtained by subjecting an n-butene-free, ethene-containing feed mixture to a second oligomerization conducted separately from the first oligomerization are added in a controlled manner to the butene oligomers to be hydroformylated.

**2.** Process according to Claim 1, **characterized in that** the ethene oligomers which are added in a controlled manner to the butene oligomers to be hydroformylated as a function of the approximation of the actual viscosity of the ester mixture determined are obtained by subjecting an n-butene-free, ethene- and hexene-containing feed mixture to the second oligomerization conducted separately from the first oligomerization, such that the second oligomerization is a co-oligomerization of ethene and hexene.

**3.** Process according to Claim 2, **characterized in that**

a) the hexene is sourced from a third oligomerization which is conducted separately from the first and second oligomerizations, in which propene is dimerized,
or
b) the hexene is sourced from a third oligomerization which is conducted separately from the first and second oligomerizations, in which ethene is trimerized;
or
c) the hexene is sourced from the second oligomerization.

**4.** Process according to Claim 1, 2 or 3, **characterized in that** the second oligomerization is effected in the presence of a liquid inert solvent in which the ethene is at least partly dissolved and which is separated from the ethene oligomers after the second oligomerization has been conducted.

**5.** Process according to any of Claims 1 to 4, **characterized in that** the controlled addition of the ethene oligomers to the butene oligomers to be hydroformylated is effected in such a way that a target viscosity of the ester mixture is first fixed, the approximation of the actual viscosity of the ester mixture determined is compared with the target viscosity of the ester mixture, and the amount of the ethene oligomers added to the butene oligomers to be hydroformylated is increased if the approximation of the actual viscosity is greater than the target viscosity, or the amount of the ethene oligomers added to the butene oligomers to be hydroformylated is reduced if the approximation of the actual viscosity is less than the target viscosity.

**6.** Process for preparing an ester mixture in which an n-butene-containing feed mixture having a composition which varies over time is subjected to a first oligomerization to obtain an oligomerizate and at least some of the butene oligomers present in the oligomerizate are hydroformylated to aldehydes, of which at least some are hydrogenated by subsequent hydrogenation to an alcohol mixture which is then converted to an ester mixture, and in which an approximation of the actual viscosity of the ester mixture is determined,
**characterized in that**
as a function of the approximation of the actual viscosity of the ester mixture determined, ethene is added in a controlled manner to the n-butene-containing feed mixture, such that the first oligomerization is a co-oligomerization of ethene and n-butene.

**7.** Process according to Claim 6, **characterized in that** controlled addition of the ethene to the feed mixture is effected in such a way that a target viscosity of the ester mixture is first fixed, the approximation of the actual viscosity of the ester mixture determined is compared with the target viscosity of the ester mixture, and the amount of the ethene added to the feed mixture is increased if the approximation of the actual viscosity is greater than the target viscosity, or the amount of ethene added to the feed mixture is reduced if the approximation of the actual viscosity is less than the target viscosity.

8. Process according to any of Claims 1 to 7,
**characterized in that** the approximation of the actual viscosity of the ester mixture is determined by measuring the viscosity of the ester mixture.

9. Process according to any of Claims 1 to 7,
**characterized in that** the approximation of the actual viscosity of the ester mixture is determined by calculation, the calculation being based on analysis of the alcohol mixture, on an analysis of the aldehydes to be hydrogenated, or on an analysis of the oligomers to be hydroformylated.

10. Process according to any of Claims 1 to 9,
**characterized in that**
the n-butene-containing feed mixture is composed of a combination of the following substance mass flow rates which vary within the specified substance mass flow rate ranges with a respective rate of variation within the specified range of rates of variation:

| Substance | Substance mass flow rate | Rate of variation |
|---|---|---|
| Isobutene: | 0 kg/s to 1 kg/s | -0.05 $g/s^2$ to 0.05 $g/s^2$ |
| 1-Butene: | 0 kg/s to 6 kg/s | -0.30 $g/s^2$ to 0.30 $g/s^2$ |
| 2-Butene (cis + trans): | 1 kg/s to 13 kg/s | -0.30 $g/s^2$ to 0.30 $g/s^2$ |
| Isobutane: | 0 kg/s to 3 kg/s | -0.15 $g/s^2$ to 0.15 $g/s^2$ |
| n-Butane: | 1 kg/s to 7 kg/s | -0.30 $g/s^2$ to 0.30 $g/s^2$ |
| Other materials: | 0 kg/s to 1 kg/s | -0.05 $g/s^2$ to 0.05 $g/s^2$ |

11. Process according to Claim 10,
**characterized in that**
the ethene used in a controlled manner is sourced from an ethene source which delivers a combination of the following substance mass flow rates which vary within the specified substance mass flow rate ranges with a respective rate of variation within the specified range of rates of variation:

| Substance | Substance mass flow rate | Rate of variation |
|---|---|---|
| Ethene: | 0.1 kg/s to 5 kg/s | -0.01 $g/s^2$ to 0.01 $g/s^2$ |
| n-Butene: | 0 kg/s to 0.01 kg/s | -0.01 $g/s^2$ to 0.01 $g/s^2$ |
| Other materials: | 0 kg/s to 1 kg/s | -0.05 $g/s^2$ to 0.05 $g/s^2$ |

12. Process according to any of Claims 1 to 11,
**characterized in that** the first oligomerization and/or the second and/or the third oligomerization is effected in the presence of a heterogeneous catalyst containing nickel, silicon and aluminium.

13. Process according to any of Claims 1 to 12,
**characterized in that** the hydroformylation is conducted in the presence of a homogeneous catalyst system comprising
either cobalt or rhodium.

14. Process according to any of Claims 1 to 13,
**characterized in that** the conversion of the alcohol mixture to the ester mixture is effected by esterifying the alcohol mixture with a carboxylic acid or carboxylic anhydride, or by obtaining the ester mixture by means of a transesterification of an existing ester with the alcohol mixture.

**15.** Process according to Claim 14, **characterized in that** the alcohol mixture is a mixture of isomeric nonyl alcohols which is reacted with phthalic acid or phthalic anhydride to give diisononyl phthalate.

## Revendications

**1.** Procédé de fabrication d'un mélange d'esters, selon lequel un mélange de départ contenant du n-butène, dont la composition se modifie avec le temps, est soumis à une première oligomérisation avec obtention d'un produit oligomérisé et au moins une partie des oligomères de butène contenus dans le produit oligomérisé sont oxydés dans une hydroformylation en aldéhydes, parmi lesquels au moins une partie est hydrogénée par hydrogénation ultérieure en un mélange d'alcools, qui est ensuite transformé en le mélange d'esters, et selon lequel une approximation de la viscosité réelle du mélange d'esters est déterminée,
**caractérisé en ce que**
en fonction de l'approximation déterminée de la viscosité réelle du mélange d'esters, des oligomères d'éthène sont ajoutés de manière ciblée aux oligomères de butène à oxyder, qui sont obtenus en soumettant un mélange de départ contenant de l'éthène et exempt de n-butène à une deuxième oligomérisation réalisée séparément de la première oligomérisation.

**2.** Procédé selon la revendication 1, **caractérisé en ce que** les oligomères d'éthène ajoutés de manière ciblée aux oligomères de butène à oxyder en fonction de l'approximation déterminée de la viscosité réelle du mélange d'esters sont obtenus en soumettant un mélange de départ contenant de l'éthène et de l'hexène et exempt de n-butène à une deuxième oligomérisation réalisée séparément de la première oligomérisation, de telle sorte que la deuxième oligomérisation soit une co-oligomérisation d'éthène et d'hexène.

**3.** Procédé selon la revendication 2, **caractérisé en ce que**

a) l'hexène est extrait d'une troisième oligomérisation réalisée séparément de la première et de la deuxième oligomérisation, lors de laquelle du propène est dimérisé,
ou
b) l'hexène est extrait d'une troisième oligomérisation réalisée séparément de la première et de la deuxième oligomérisation, lors de laquelle de l'éthène est trimérisé ;
ou
c) l'hexène est extrait de la deuxième oligomérisation.

**4.** Procédé selon la revendication 1, 2 ou 3, **caractérisé en ce que** la deuxième oligomérisation a lieu en présence d'un solvant inerte liquide dans lequel l'éthène est au moins partiellement dissous et qui est séparé des oligomères d'éthène après la réalisation de la deuxième oligomérisation.

**5.** Procédé selon l'une quelconque des revendications 1 à 4, **caractérisé en ce que** l'ajout ciblé des oligomères d'éthène aux oligomères de butène à oxyder a lieu de telle sorte qu'une viscosité de consigne du mélange d'esters est tout d'abord fixée, l'approximation déterminée de la viscosité réelle du mélange d'esters est comparée avec la viscosité de consigne du mélange d'esters, et la quantité d'oligomères d'éthène ajoutés aux oligomères de butène à oxyder est augmentée lorsque l'approximation de la viscosité réelle est plus grande que la viscosité de consigne ; ou la quantité des oligomères d'éthène ajoutés aux oligomères de butène à oxyder est réduite lorsque l'approximation de la viscosité réelle est plus petite que la viscosité de consigne.

**6.** Procédé de fabrication d'un mélange d'esters, selon lequel un mélange de départ contenant du n-butène, dont la composition se modifie avec le temps, est soumis à une première oligomérisation avec obtention d'un produit oligomérisé, et au moins une partie des oligomères de butène contenus dans le produit oligomérisé sont oxydés dans une hydroformylation en aldéhydes, parmi lesquels au moins une partie est hydrogénée par hydrogénation ultérieure en un mélange d'alcools, qui est ensuite transformé en le mélange d'esters, et selon lequel une approximation de la viscosité réelle du mélange d'esters est déterminée, **caractérisé en ce que**
en fonction de l'approximation déterminée de la viscosité réelle du mélange d'esters, de l'éthène est ajouté de manière ciblée au mélange de départ contenant du n-butène, de telle sorte que la première oligomérisation soit une co-oligomérisation d'éthène et de n-butène.

**7.** Procédé selon la revendication 6, **caractérisé en ce que** l'ajout ciblé d'éthène au mélange de départ a lieu de telle sorte qu'une viscosité de consigne du mélange d'esters est tout d'abord fixée, l'approximation déterminée de la viscosité réelle du mélange d'esters est comparée avec la viscosité de consigne du mélange d'esters, et la quantité d'éthène ajouté au mélange de départ est augmentée lorsque l'approximation de la viscosité réelle est plus grande que la viscosité de consigne ; ou la quantité d'éthène ajouté au mélange de départ est réduite lorsque l'approximation de la viscosité réelle est plus petite que la viscosité de consigne.

**8.** Procédé selon l'une quelconque des revendications 1 à 7, **caractérisé en ce que** l'approximation de la viscosité réelle du mélange d'esters est déterminée par mesure de la viscosité du mélange d'esters.

**9.** Procédé selon l'une quelconque des revendications 1 à 7, **caractérisé en ce que** l'approximation de la viscosité réelle du mélange d'esters est déterminée par calcul, le calcul ayant lieu au choix à partir d'une analyse du mélange d'alcools, à partir d'une analyse des aldéhydes à hydrogéner ou à partir d'une analyse des oligomères à oxyder.

**10.** Procédé selon l'une quelconque des revendications 1 à 9, **caractérisé en ce que** le mélange de départ contenant du n-butène se compose d'une combinaison des débits massiques de substances suivants, qui se modifient dans les plages de débits massiques de substances indiquées avec une vitesse de modification respective qui se situe dans la plage de vitesse de modification indiquée :

| Substance | Débit massique de substance | Vitesse de modification |
|---|---|---|
| Isobutène : | 0 kg/s à 1 kg/s | -0,05 $g/s^2$ à 0,05 $g/s^2$ |
| 1-Butène : | 0 kg/s à 6 kg/s | -0,30 $g/s^2$ à 0,30 $g/s^2$ |
| 2-Butène (cis + trans) : | 1 kg/s à 13 kg/s | -0,30 $g/s^2$ à 0,30 $g/s^2$ |
| Isobutane : | 0 kg/s à 3 kg/s | -0,15 $g/s^2$ à 0,15 $g/s^2$ |
| n-Butane : | 1 kg/s à 7 kg/s | -0,30 $g/s^2$ à 0,30 $g/s^2$ |
| Autres substances : | 0 kg/s à 1 kg/s | -0,05 $g/s^2$ à 0,05 $g/s^2$ |

**11.** Procédé selon la revendication 10, **caractérisé en ce que** l'éthène ajouté de manière ciblé est extrait d'une source d'éthène qui fournit une combinaison des débits massiques de substances suivants, qui se modifient dans les plages de débits massiques de substances indiquées avec une vitesse de modification respective qui se situe dans la plage de vitesse de modification indiquée :

| Substance | Débit massique de substance | Vitesse de modification |
|---|---|---|
| Éthène : | 0,1 kg/s à 5 kg/s | -0,01 $g/s^2$ à 0,01 $g/s^2$ |
| n-Butène : | 0 kg/s à 0,01 kg/s | -0,01 $g/s^2$ à 0,01 $g/s^2$ |
| Autres substances : | 0 kg/s à 1 kg/s | -0,05 $g/s^2$ à 0,05 $g/s^2$ |

**12.** Procédé selon l'une quelconque des revendications 1 à 11, **caractérisé en ce que** la première oligomérisation et/ou la deuxième et/ou la troisième oligomérisation ont lieu en présence d'un catalyseur hétérogène, qui contient du nickel, du silicium et de l'aluminium.

**13.** Procédé selon l'une quelconque des revendications 1 à 12, **caractérisé en ce que** l'hydroformylation a lieu en présence d'un système catalytique homogène, qui comprend du cobalt ou du rhodium.

**14.** Procédé selon l'une quelconque des revendications 1 à 13, **caractérisé en ce que** la transformation du mélange d'alcools en le mélange d'esters a lieu de telle sorte que le mélange d'alcools est estérifié avec un acide carboxylique ou un anhydride d'acide carboxylique, ou que le mélange d'esters est obtenu par une transestérification d'un ester existant avec le mélange d'alcools.

**15.** Procédé selon la revendication 14, **caractérisé en ce que** le mélange d'alcools est un mélange d'alcools nonyliques isomères, qui est transformé avec de l'acide phtalique ou de l'anhydride d'acide phtalique en phtalate de diisononyle.

visc
C4
Oli
2C4
HyFo
C9-al
Hydro
C9-ol
Esterfication
Ester
H2 + CO
H2
Acid

Fig.0
(prior art)

C4
Oli I
2C4
C2
Oli II
4C2
C8
HyFo
C9-al
Hydro
C9-ol
Esterfication
Ester
visc
H2 + CO
H2
Acid

Fig.1

C4
Oli I
2 C4
C8
HyFo
C9-al
Hydro
C9-ol
Esterfication
Ester
visc
C2
C2 + C6
Co-Oli
C8
C6
H2 + CO
H2
Acid

Fig.2

C4
Oli
2C4
C8
HyFo
C9-al
Hydro
C9-ol
Esterfication
Ester
visc
C2
C2 + C6
Co-Oli
C8
H2 + CO
H2
Acid
C2
3C2
Oli III


Fig.3

C4
Oli
2C4
C8
HyFo
C9-al
Hydro
C9-ol
Esterfication
Ester
visc
C2
C2 + C6
Co-Oli
C8
H2 + CO
H2
Acid
2C3
C3
Oli III

Fig.4

visc
C4
C2+C4
Co-Oli
C8
HyFo
C9-al
Hydro
C9-ol
Esterfication
Ester
C2
H2 + CO
H2
Acid

Fig.5

C4
Oli I
2C4
C8
HyFo
C9-al
Hydro
C9-ol
Esterfication
Ester
visc
4C2
C2
C2, Solv
Oli II
4C2, Solv
H2 + CO
H2
Acid
Solv

Fig.6

C4
Oli I
2C4
C8
HyFo
C9-al
Hydro
C9-ol
Esterfication
Ester
visc
C2
C2, 3C2
Co-Oli
4C2, 3C2
4C2
H2 + CO
H2
Acid
3C2
C6-Excess

Fig.7

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102008007081 A1
- EP 1029839 A1
- WO 2014131623 A
- EP 1219584 B1
- EP 1186593 B1
- EP 1300388 B1
- EP 1430014 B1
- US 6433242 B1
- WO 2014207034 A1
- EP 15168100 A
- US 14717183 B
- EP 15151621 A
- US 2581228 A


### In der Beschreibung aufgeführte Nicht-Patentliteratur


- Butenes. **GEILEN, F. M. ; STOCHNIOL, G. ; PEITZ, S. ; SCHULTE-KOERNE, E.** Ullmann's Encyclopedia of Industrial Chemistry. 31. Januar 2014
- **R. H. FRIEDLANDER ; D. J. WARD ; F. OBENAUS ; F. NIERLICH ; J. NEUMEISTER.** Make plasticizer olefins via n-butene dimerization. *Hydrocarbon Processing,* Februar 1986, 31-33
- **R. FRANKE ; D. SELENT ; A. BÖRNER.** Applied Hydroformylation. *Chem. Rev.,* 2012, vol. 112, 5675-5732
- **J. FALBE ; H. BAHRMANN ; W. LIPPS ; D. MAYER ; G. D. FREY.** Alcohols Aliphatic in Ullmann's Encyclopedia of Industrial Chemistry. 2013
- **D. SANFILIPPO ; P. N. RYLANDER.** Hydrogenation and Dehydrogenation in Ullmann's Encyclopedia of Industrial Chemistry. 2009